# EUROPEAN PATENT SPECIFICATION

(11) **EP 1 633 465 B1**
(45) Date of publication and mention of the grant of the patent: **26.11.2008**
(21) Application number: 04754103.2
(22) Date of filing: 02.06.2004
(51) Int. Cl.: B01F 5/10, B01F 13/08, B01F 15/00

(54) **MIXING BAG FOR POWDER, APPARATUS, METHOD AND SYSTEM FOR USING SAME**
MISCHBEUTEL FÜR PULVER; VORRICHTUNG, VERFAHREN UND SYSTEM ZUR VERWENDUNG DAVON
SAC DE MELANGE POUR UNE POUDRE, DISPOSITIF, PROCEDE ET SYSTEME DESTINES A L'UTILISATION DE CE SAC DE MELANGE

(30) Priority: 03.06.2003 US 454173; 03.06.2003 US 475658 P; 28.05.2004 US 857479
(43) Date of publication of application: 15.03.2006
(73) Proprietor: Advanced Minerals Corporation, Santa Barbara, California 93117 (US)
(72) Inventor: HURST, William, E., Santa Barbara, CA 93105 (US)
(74) Representative: Nash, David Allan
(86) International application number: PCT/US2004/017416
(87) International publication number: WO 2004/108262

(56) References cited:
- EP-A- 0 619 135
- DE-A- 10 100 549

## Description

### CROSS-REFERENCES TO RELATED PATENT APPLICATIONS

This application claims Convention priority and priority under 35 U.S.C. § 119(e) to U.S. Patent Application No. 60/475,658 (fled 3 June 2003 and entitled "Preformed Mixing Bag for Dry Powder, Apparatus, Method and System for Using Same").

This application claims Convention priority to and is a U.S. continuation-in-part of U.S. Patent Application No. 10/454,173 (filed 3 June 2003 and entitled "Preformed Mixing Bag for Dry Powder, Apparatus, Method and System for Using Same").

This application claims Convention priority to and is a U.S. continuation-in-part of a new U.S. patent application that was filed on 28 May 2004 and that is entitled "Preformed Mixing Bag for Dry Powder, Apparatus, Method and System for Using Same." (The serial number for this new application is not yet known.).

### BACKGROUND

### 1. Field:

The present invention relates to a method comprising the use of a defined mixing bag for packaging, storing and shipping (using a separate shipping container) a dry material to be mixed in the mixing bag; more particularly the method of the invention comprises the use of a defined preformed mixing bag adapted to store a dry powder which is to be mixed with a process compatible solution to form a suspension of powder in the solution having the material in suspension with a selected concentration which may be at least one of a high concentration capable of being diluted to a target concentration or at a target concentration intended for use without being diluted.

### 2. Description of the Art

The use of mixing bags for storing a dry powder to be mixed in the mixing bag with a liquid is known in the art. The following prior art discloses typical mixing bags, apparatus and method for using such mixing bags.

United States Patent 5,616,305 discloses a bag containing a selected quantity of dry powdered material wherein the volume of the bag is sufficient to add a liquid of a known maximum volume resulting in a solution in the form of a powdered salt concentrate wherein the solution is mixed by use of a pump connected to a water supply line. The powdered salt partially dissolves when the bag is completely filled with water.

United States Patent 5,643,585 discloses a non-soluble articulate coloring material comprising dry ground red micro algae-derived material as a base color material. The non-soluble particulate matter was stored in a transparent plastic bag for testing as opposed to mixing with a fluid.

United States Patents 6,149,294; 5,511,875 and 5,348,389 each disclose a system for mixing a powder and a fluid to produce a slurry. The system has a bag for mixing the powder and fluid therein and discloses use of a pump that recirculates and mixes the contents of the bag.

United States Patent 5,362,642 discloses a cell culture media containment system having a mixing bag substantially enclosed within a storage bag powdered cell culture media and other constituents are introduced into the mixing bag and are mixed therein. Therefore, the reconstituted media is conveyed from the mixing bag into the storage bag. The mixing bag has an access port in the top to assist in the introduction of constituents into the mixing chamber and an introduction plate in the top having an opening allowing for insertion of tubes there through into the mixing chamber. The storage bag has an introduction plats having an opening for possessing tubing into the storage bag and to support a discharge tube.

United States Patent 5,121,857 discloses a system for agitating and dispensing a mixture from a bag-in-box arrangement wherein the bag-in-box has a connector having an inlet port, an outlet port and a pump.

United States Patent 5,709,467 discloses a bag or a pouch for mixing and dispensing an alginate and water composition and wherein the pouch or bag as a sealable opening.

United States Patent 6,298,984 discloses a package containing a medium that must be combined with an activator and mixed before the medium can be used by an end user.

United States Patent 4,948,013 discloses a method of mixing a powder and a fluid wherein a filter is used to allow only predetermined particles to be dispensed.

United States Patents 5,069,370 and 4,821,923 each disclose mixing and dispensing containers.

United States Design Patent Number DES 413,258 discloses a flexible bag in the form of a dunnage bag having a valve, the dunnage bag apparently being used for personal storage of fluids or personal belongings.

United States Design Patent Number DES 308,164 discloses a flexible container having an extended pouring spout for storing and dispensing fluids.

United States Design Patent Number DES 304,546 discloses a flexible container having an opening which appears to be used for a filling the container with a fluid causing expansion of a bottom seam to accommodate a pre-determined volume fluid and wherein the opening is used to dispense the fluid.

European Patent Office EPO Publication Number 0 152 283 discloses apparatus for dispensing a beverage from a reservoir wherein the reservoir containing the fluid is releasably connected to the apparatus wherein a pump continuously circulates the beverage around a fluid circuit. One or more dispensing valves or taps are located in the fluid circuit from which the beverage is to be dispensed. A metering pump is provided to effect mixing of the beverage within the reservoir when the beverage is to be formed of two or more liquids.

European Patent Office EPO Publication Number 0 354 685 discloses a system for agitating and dispensing a fluid in a bag-in box arrangement and appears to be a foreign equivalent of United States Patent 5,121,857 discussed above.

Discloses a system for preparation and use of dialyses solution United States Patent 5,3-85,564. Having a bag which is constructed to hold granulated or powder concentrate. The bag has a V-shaped bottom with an access port in the bottom. Water is flowed through the access port and into the bag using water control means having a pump. The incoming water lifts and suspends the granules or powder. The filling of the bag is monitored by trip switches operatively attached to support the weight of the bag and contents. When the bag is filled sufficiently with water, the weight of the bag and contents trips the trip switches and a signal is sent to deactivate the pumps through control circuits.

DE-A-10100549 discloses a salt concentrate container for dialysis apparatus, with 2 openings connected to a liquid circuit, wherein the salt concentrate is soluble in the dialysis liquid. EP-A-0619135 discloses a system for preparing a fluid for medical treatment by dissolving a first compound (such as a saline concentrate) in a second compound (such as an haemodialysis fluid), the system comprising the use of a specific ultrasound generator for assisting dissolution.

More specifically, the document EP-A-0619135 discloses a method comprising the use of a mixing bag having an interior mixing chamber having a first end located in a top section and a second end located in a bottom section and having a predetermined weight of a first compound (such as saline concentrate), said mixing bag having:
- a first sealable opening extending from a location exterior to the mixing bag and into communication with the interior mixing chamber at said first end and which is configured for at least one of enabling transportation of the first compound into the interior mixing chamber, enabling agitation of the first compound with a second compound (such as water) in the interior mixing chamber to form a solution and enabling withdrawal of the solution from the interior mixing chamber;
- a second sealable opening extending from a location exterior to the mixing bag and into communication with the interior mixing chamber at said second end and which is configured for at least one of enabling injection of the second compound into the interior mixing chamber to form a solution, enabling agitation of the first and second compounds in the interior mixing chamber to form a solution and enabling withdrawal of the solution from the interior mixing chamber; and
- a third sealable opening extending from a location exterior to the mixing bag and into communication with the interior mixing chamber and which is configured for enabling transporting the first compound into the interior mixing chamber.

### BRIEF SUMMARY

The present invention is directed to a method comprising the use of a mixing bag, as defined in claim 1. The method of the invention enables mixing a dry powder with a process compatible solution in a preformed mixing bag to form a suspension of powder in the process compatible solution having the material in suspension with a selected concentration which is at least one of a high concentration capable of being diluted to a target concentration and a target concentration or, in the alternative, a selected density or a density within or acceptable range of densities. The mixing bag, which may be a preformed mixing bag, comprises an interior mixing chamber having a volume sufficient to receive a predetermined weight of dry powder and a selected volume of process compatible solution to form a solution having powder in suspension with a selected concentration, e.g. g/L or selected density e.g. g/L³. The selected concentration is at least one of a high concentration capable of being diluted to a target concentration and a target concentration.

The dry powder may be for example a Celpure^{®} media or e.g. a slightly hydrated media such as for example an LRA^{®} media.

The selected concentration may be at a high concentration of Celpure^{®} media or LRA^{®} media, e.g. 100 g/L, to minimize the size of the system and the resulting solution may then be diluted with an inline mixer to achieve a lower target concentration, e.g. 2g/L.

Therefore, it is an advantage of the present invention to provide the use of a mixing bag having a volume sufficient to receive a predetermined weight of dry material capable of being forming and remaining in suspension in a process compatible solution and a selected volume of process compatible solution to form a solution having the material in suspension with a selected concentration or selected solution density.

Another advantage of the present invention is that the mixing bag can be used in methods, apparatus and a system for forming a solution having the material in suspension with a selected concentration which is at least one of a high concentration capable of being diluted to a target concentration and a target concentration intended for use without being diluted.

Another advantage of the present invention is that the selected concentration of the solution may be a high concentration to minimize system size and the resulting solution may then be diluted to achieve a lower target concentration.

Another advantage of the present invention is that a mixing bag can be a preformed mixing bag having a selected shape.

Another advantage of the present invention is that the mixing bag can be have a volume sufficient to receive a predetermined weight of dry material capable of being forming and remaining in suspension in a. process compatible solution and a selected volume of a process compatible solution to form a solution having the material in suspension with a selected concentration.

Another advantage of the present invention is that the mixing bag can be have a volume sufficient to receive a predetermined weight of non-soluble powder capable of being forming and remaining in suspension in a process compatible solution and a selected volume of process compatible solution to form a solution having the material in suspension with a selected concentration. In the preferred embodiment, the non-soluble powder is diatomaceous earth. In alternative applications, the non-soluble powder may comprise Celpure^{®} media, LRA^{®} media or sorbents.

Another advantage of the present invention is that the mixing bag can have an outer member having a predetermined shape and a sidewall defining an interior mixing chamber having a volume sufficient to receive a predetermined weight of dry powder and a selected volume of process compatible solution to form a solution.

Another advantage of the present invention is that the mixing bag can have an outer member which is in the shape of an elongated thin walled rectangular member having a hollowed-out passageway defining the interior mixing chamber.

Another advantage of the present invention is that the mixing bag can have an outer member which is in the shape of an elongated thin walled cylindrical member having a hollowed-out passageway defining the interior mixing chamber.

Another advantage of the present invention is that the mixing bag can have an outer member which is in the shape of an elongated thin walled cylindrical member having a hollowed-out passageway defining the interior mixing chamber wherein the interior mixing chamber includes an internal conical shaped hopper for receiving dry powder.

Another advantage of the present invention is that the mixing bag comprises a third sealable opening which is configured for enabling transporting dry powder into the interior mixing chamber; a second sealable which is configured for at least one of enabling injection of process compatible solution into the interior mixing chamber to form a solution having the powder in suspension, enabling agitation of the powder and process compatible solution in the interior mixing chamber to form a solution having the powder in suspension with a selected concentration and enabling withdrawal of the solution with a selected concentration from the interior mixing chamber; and a first sealable opening which is configured for enabling at least one of agitation of the powder and process compatible solution in the interior mixing chamber to form a solution having the powder in suspension with a selected concentration, and enabling with drawal of the solution with a selected concentration from the interior mixing chamber.

The method of the invention comprises the steps of injecting a selected volume of process compatible solution, e.g. water or feedstock solution, into a mixing bag through the second sealable opening; agitating through at least one of said first and second sealable openings the powder and process compatible solution in the interior mixing chamber to form a solution having powder in suspension with a selected concentration and withdrawing from the interior mixing chamber through at least on of said first and second sealable openings the solution with a selected concentration. The selected concentration is at least one of a high concentration capable of being diluted to a target concentration and a target concentration intended for use without being diluted.

Another advantage of the present invention is that the mixing bag having an interior mixing chamber having a predetermined weight of dry powder, e.g. a predetermined volume of diatomaceous earth, can be fabricated as a bioprocess container having three sealable inlets or openings. The three sealable openings can be used for a number of functions. The second sealable opening can be used for injecting a process compatible solution and/or a feedstock solution into the interior mixing chamber. As such, a selected volume of process compatible solution, e.g. make-up water or feedstock solution, can be injected into the container to form a solution comprising diatomaceous earth in suspension with a selected concentration, or selected solution density or a solution density which is within a density range of acceptable solution densities. The first and second sealable inlets or openings can be used to provide fluid flow for agitating or mixing the solution, for having a vent filter placed in line or for withdrawing solution with a selected concentration, or selected solution density or a solution density which is within a density range of acceptable solution densities. The selected solution density and density range of the solution and batch mixing specifications are determined by the requirements of the end user. The first and second sealable openings are provided to with draw the solution from the mixing bag.

### BRIEF DESCRIPTION OF THE DRAWINGS

The present invention will become more fully understood from the following detailed description of a preferred but nonlimiting embodiment thereof, described in connection with the accompanying drawings, wherein:

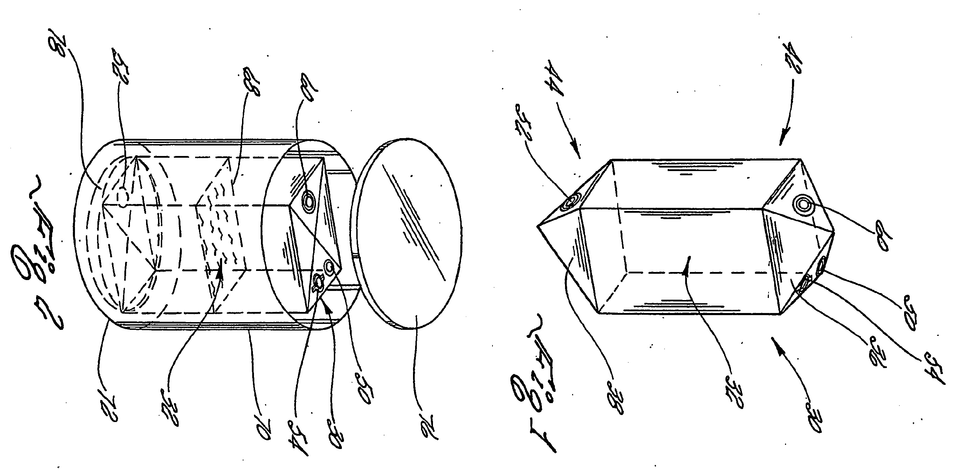

Fig. 1 is a front, left side and top perspective view of a preformed mixing bag, having a top section and a bottom section, adapted to store a dry material, e.g. dry powder in an interior mixing chamber which is to be mixed with a process compatible solution to form a suspension of powder in the solution having the material in suspension with a selected concentration and wherein the preformed mixing bag has at least two sealable openings;
Fig. 2 is a perspective pictorial representation of a shipping container having placed therein a preformed mixing bag of Fig. 1 filled with a predetermined weight of dry powder for shipping to an end user for subsequent injection of a process compatible solution into the interior mixing chamber to form a solution having powder in suspension;

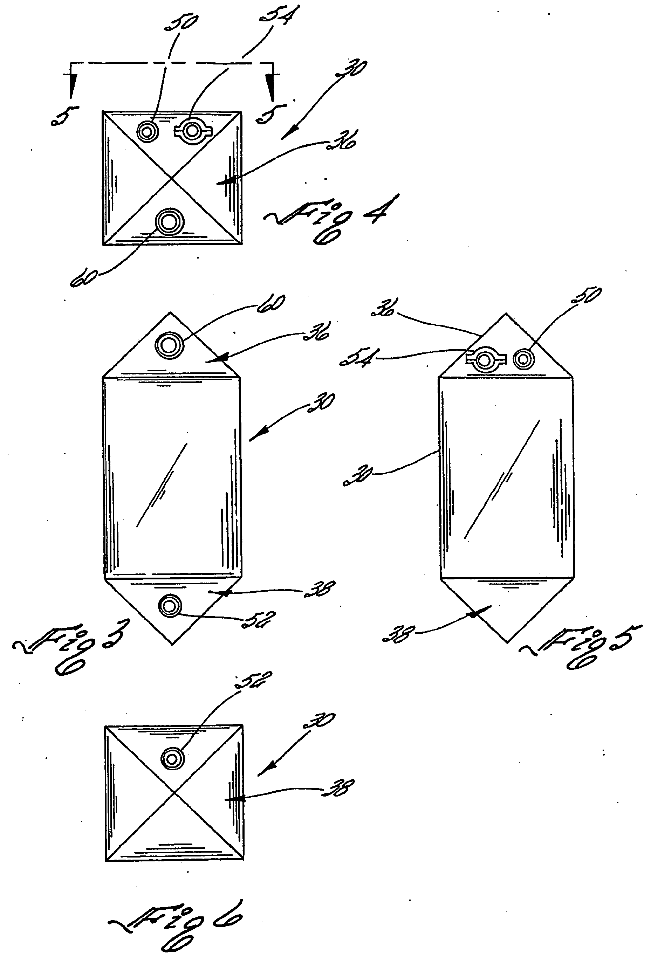

Fig. 3 is a front elevational view of a preformed mixing bag of Fig. 1 showing the second sealable opening for at least one of enabling injection of a process compatible solution into said interior mixing chamber to form a solution having powder in suspension, enabling agitation of the powder and process compatible solution within the interior mixing chamber to form a solution having powder in suspension with a selected concentration, and enabling withdrawal of the solution with a selected concentration from the interior mixing chamber;
Fig. 4 is a top plan view of the preformed mixing bag of Fig. 1 showing the third sealable opening configured for enabling transporting a predetermined weight of dry powder into the interior mixing chamber and the first sealable opening for enabling at least one of agitation of the powder and process compatible solution within the interior mixing chamber to form a solution having powder in suspension with a selected concentration and enabling withdrawal of solution from the interior mixing chamber at a selected concentration and wherein the preformed mixing bag can optionally include an auxiliary opening which can be used as required;
Fig. 5 is a back elevational view of a preformed mixing bag of Fig. 1 showing the third sealable opening configured for enabling transporting a predetermined weight of dry powder into the interior mixing chamber and the first sealable opening for at least one of enabling agitation of the powder and process compatible solution within the interior mixing chamber to form a solution having powder in suspension with a selected concentration and enabling withdrawal of solution from the interior mixing chamber at a selected concentration;
Fig. 6 is a bottom plan view of the preformed mixing bag of Fig. 1 showing the second sealable opening for at least one of enabling injection of a process compatible solution into said interior mixing chamber to form a solution having powder in suspension, enabling agitation of the powder and process compatible solution within the interior mixing chamber to form a solution having powder in suspension with a selected concentration and enabling withdrawal of solution from the interior mixing chamber at a selected concentration;

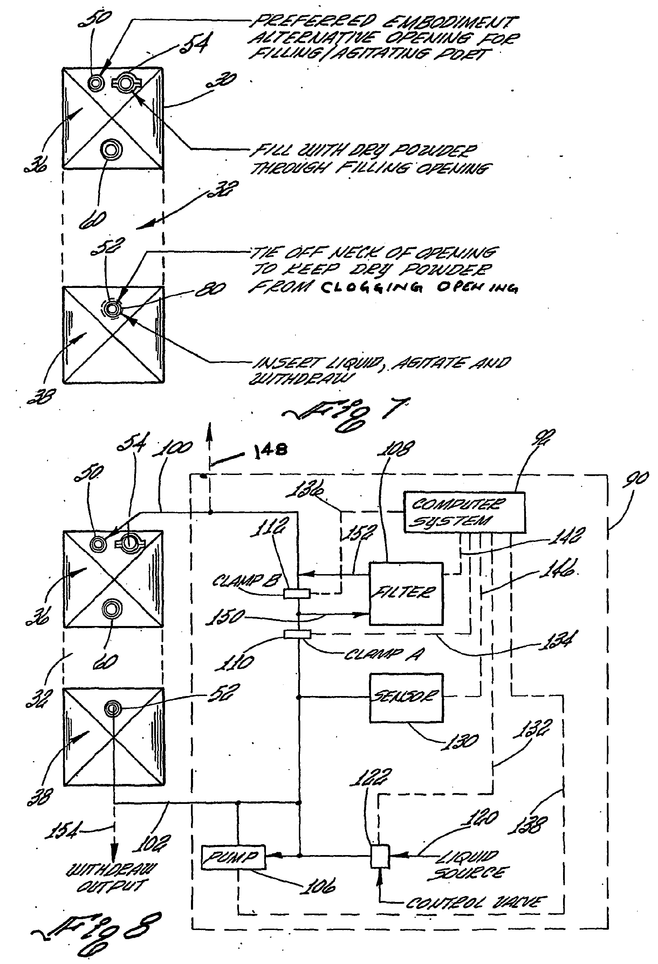

Fig. 7 is a pictorial representation of the of the mixing bag of Fig. 1 showing a preformed mixing bag having a first sealable opening, a second sealable opening, a third sealable opening and an auxiliary opening, the relationship between and functions of the first, second and third sealable openings and use of the first sealable opening for enabling transporting of a predetermined weight of dry powder into the interior mixing chamber;
Fig. 8 is a pictorial representation of the of the mixing bag of Fig. 1 showing a preformed mixing bag having a first sealable opening, a second sealable opening, a third salable opening and an auxiliary opening, the relationship between and functions.of the first, second and third sealable openings for enabling transporting a predetermined weight of dry powder into the interior mixing chamber, enabling injection of a process compatible solution into the interior mixing chamber to form a solution having powder in suspension, enabling agitation of the powder and process compatible solution to form a solution having powder in suspension with a selected concentration and enabling withdrawal of the solution from the interior mixing chamber with a selected concentration under control of a mixing control system having a computer system;

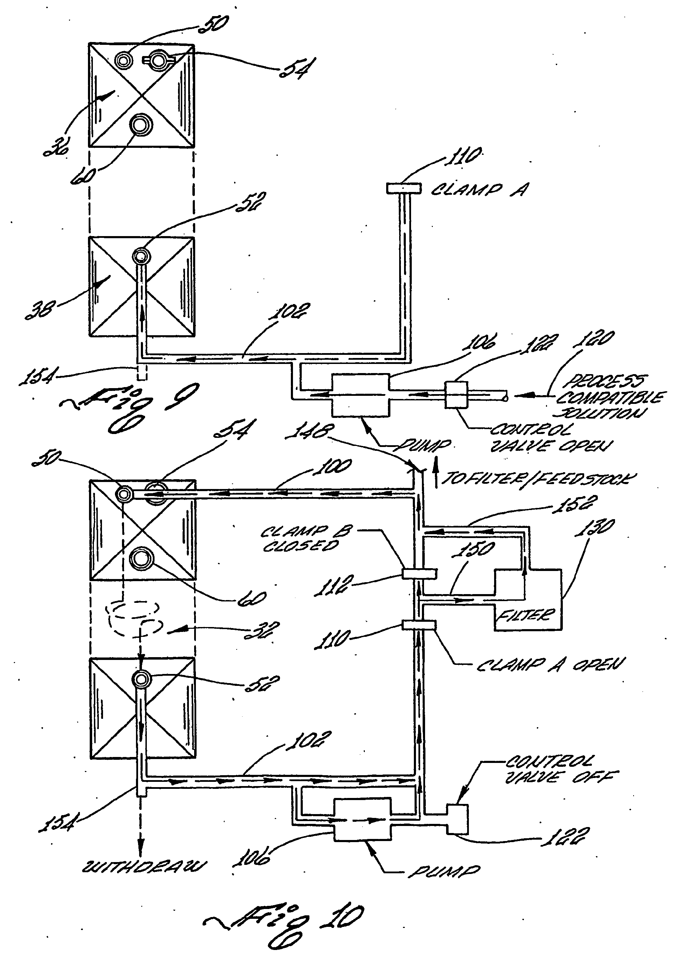

Fig. 9 is a pictorial representation of the of the mixing bag of Fig. 1 having a first sealable opening, a second sealable opening, third sealable opening and an auxiliary opening showing the use of the second salable opening for enabling injection of a process compatible solution into the interior mixing chamber through the bottom section of the mixing bag to form a solution having powder in suspension;
Fig. 10 is a pictorial representation of the of the mixing bag of Fig. 1 having a first sealable opening, a second sealable opening, a third sealable opening and an auxiliary opening showing the use of the second salable opening and the first sealable' opening for enabling agitation of the powder and process compatible solution to form a solution having powder in suspension with a selected concentration, enabling filtering of the solution and for enabling withdrawal of the solution from the interior mixing chamber with a selected concentration through a filter feedstock conduit under control of a mixing control system;

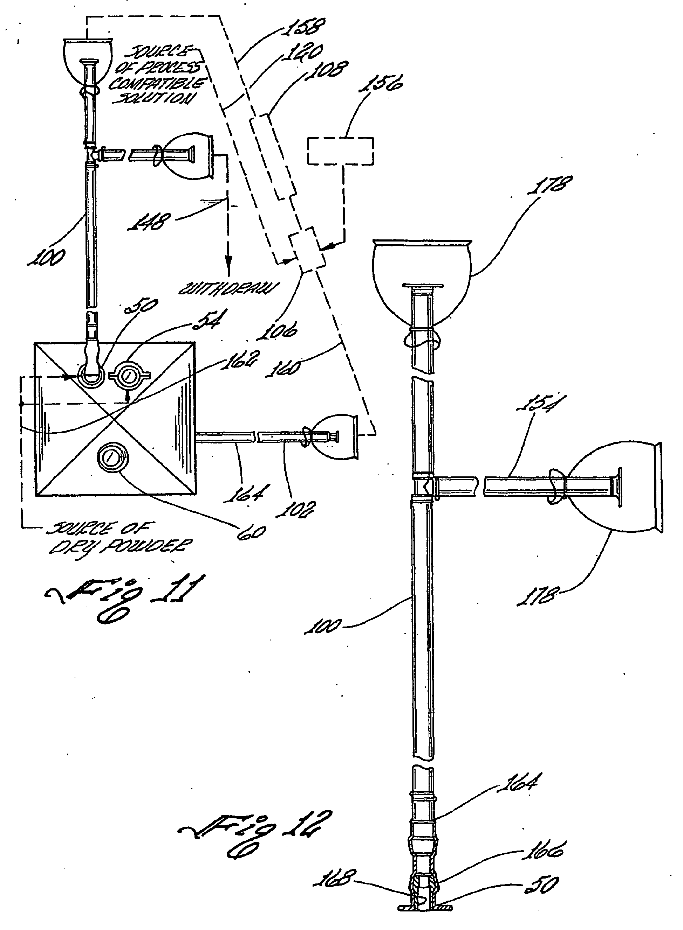

Fig. 11 is a diagrammatic representation of a mixing bag of the present invention having a source of dry powder, a first conduit system and a second conduit system including showing in dashed lines connecting hoses, a pump, a filter and a controller for controlling the injecting of make-up, process compatible solution of a selected volume into the interior mixing chamber and for withdrawing of solution from the interior mixing chamber at a selected concentration;
Fig. 12 is a top plan view of the first conduit system shown in Fig. 11 operatively removably connected to one of the first and second openings for enabling agitating the solution of powder and process compatible solution in the interior mixing chamber to form a solution of the process compatible solution having the powder in suspension with a selected concentration;

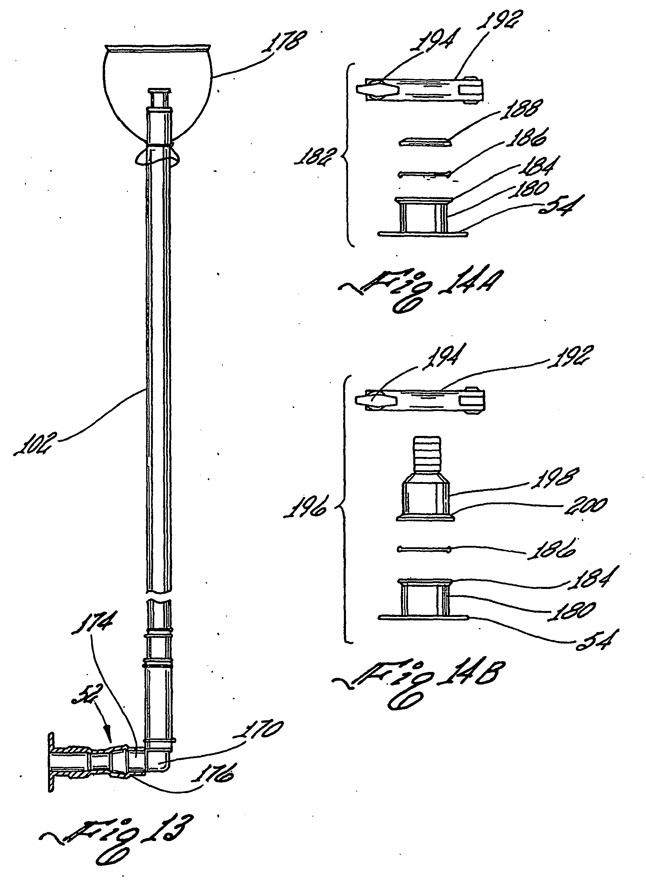

Fig. 13 is a top plan view of the second conduit system shown in Fig. 11 operatively removeably connected to the second opening for at least one of injecting process compatible solution into the interior mixing chamber to form a solution having powder in suspension, enabling agitation of the powder and process compatible solution in the interior mixing chamber to form a solution having powder in suspension with a selected concentration and in cooperation with the first conduit system enabling withdrawal of the solution with a selected concentration from the interior mixing chamber;
Fig. 14A is an exploded front elevational view of the third sealable opening having a port assembly with a sealing cap for enabling transporting a predetermined weight of dry powder into the interior mixing chamber of a mixing bag and sealing of the port assembly;
Fig. 14B is an exploded front elevational view of the third sealable opening having a port assembly with an elongated attachment member for enabling transporting a predetermined weight of dry powder into the interior mixing chamber of a mixing bag through the elongated attachment member;

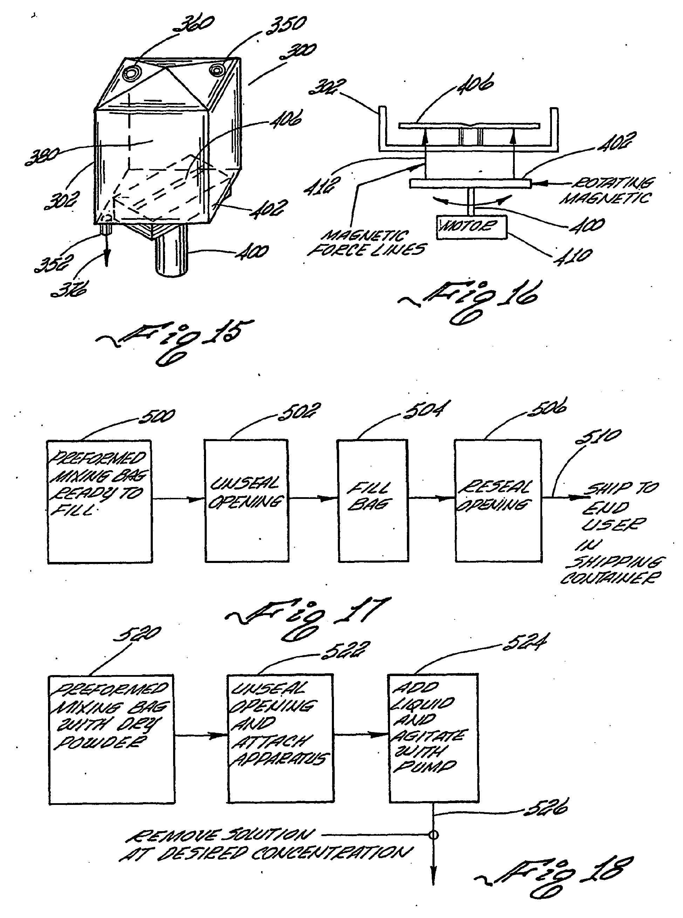

Fig. 15 is a pictorial representation of yet another embodiment or a preformed mixing bag for practicing the teachings of the present invention wherein a magnetically controlled impeller is used for agitating the solution;
Fig. 16 is a pictorial representation of a magnetically controlled impeller configured for use in the embodiment illustrated in Fig. 15;
Fig. 17 is a block diagram of the method for filling a preformed mixing bag having an interior mixing chamber configured to have a volume sufficient to receive a predetermined weight of dry powder and a selected volume of process compatible solution to form a solution having powder in suspension with a selected concentration and shipping the same in a shipping container to an end user;
Fig. 18 is a block diagram of the method for using a preformed mixing bag having an interior mixing chamber containing a predetermined weight of dry powder wherein the sealable openings are unsealed for enabling injection of a process compatible solution into the interior mixing chamber to form a solution having powder in suspension, enabling agitation of the powder and process compatible solution to form a solution having powder in suspension with a selected concentration and enabling withdrawal of the solution from the interior mixing chamber with a selected concentration or at a desired concentration;

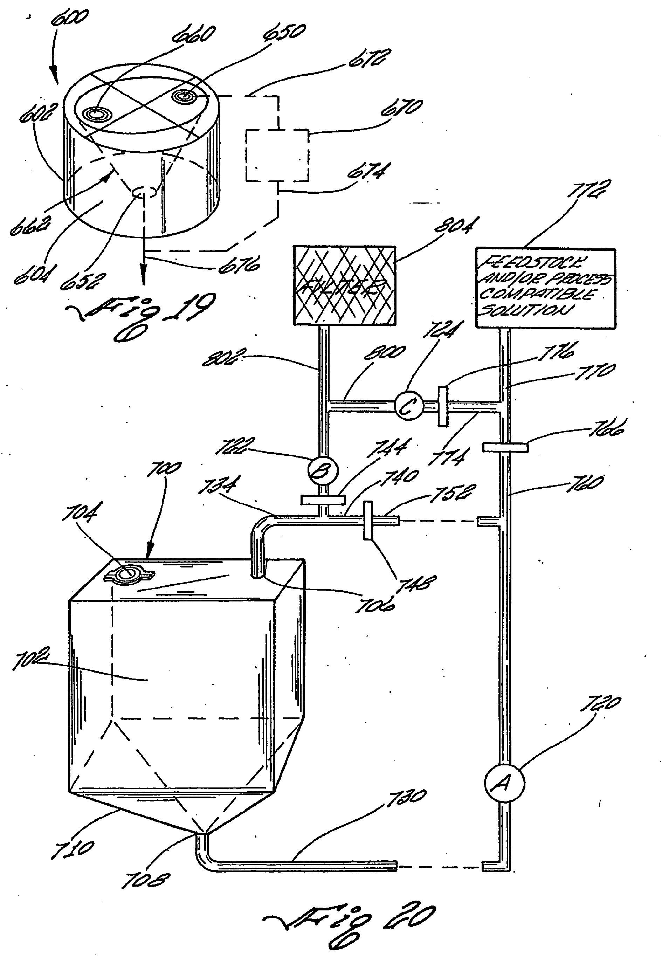

Fig. 19 is a pictorial representation of another embodiment of a preformed mixing bag having a conical shaped internal hopper for practicing the teachings of the present invention;
Fig. 20 is a pictorial representation of another embodiment of a preformed mixing bag in a system for the delivery of Celpure^{®} media to a filtration process wherein the suspension produces a Celpure^{®} media body-feed concentration produced with a process compatible solution;

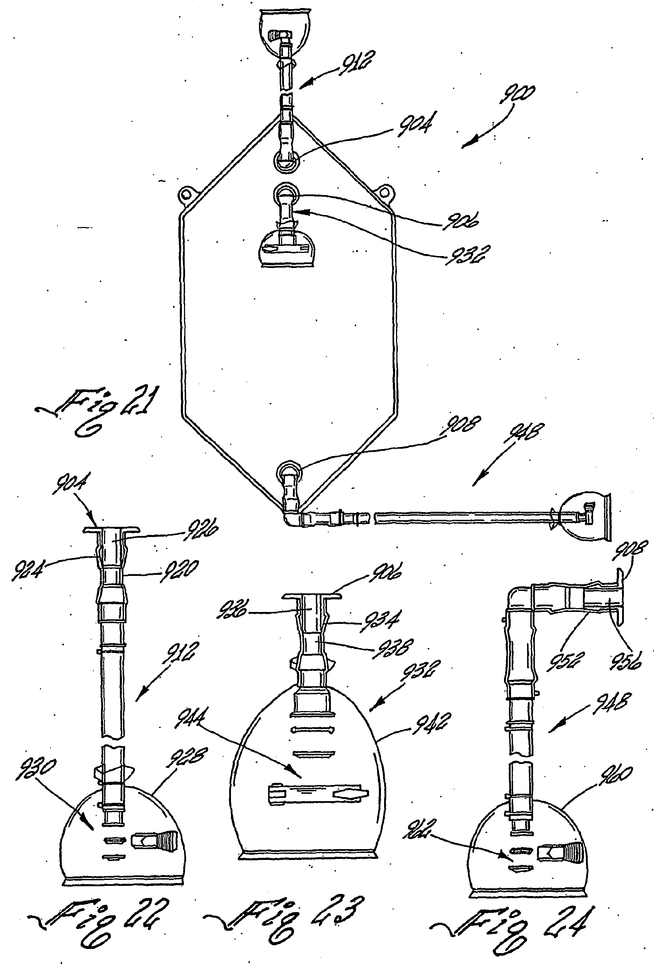

Fig. 21. is a pictorial representation of another embodiment/species of a preformed mixing bag in the form of a bioprocess container having three sealable inlets or openings used to inject a process compatible fluid (or a feedstock solution or make-up water) to be processed to form the solution, for agitating the solution and/or withdrawing the solution from the bioprocess container,
Fig. 22 is a top plan view of a first conduit system shown in Fig. 21 operatively removeably connected to the first sealable inlet or opening for agitating the solution of powder and process compatible solution in the interior mixing chamber to form a solution of the process compatible solution having the powder in suspension with a elected concentration in the presence of a feed stock material;
Fig. 23 is a top plan view of a second conduit system shown in Fig. 21 operatively removably connected to the third sealable inlet or opening for enabling filling of a dry powder into the interior of the bioprocess mixing bag,
Fig. 24 is a top plan view of a third conduit system shown in Fig. 21 operatively removeably connected to the second sealable inlet or opening for at least one of injecting process compatible solution into the interior mixing chamber to form a solution having powder in suspension, for enabling agitation of the powder and process compatible solution in the interior mixing chamber to form a solution having powder in suspension with a selected concentration and in cooperation with the first conduit system and/or enabling withdrawal of the solution with a selected concentration from the interior mixing chamber; and

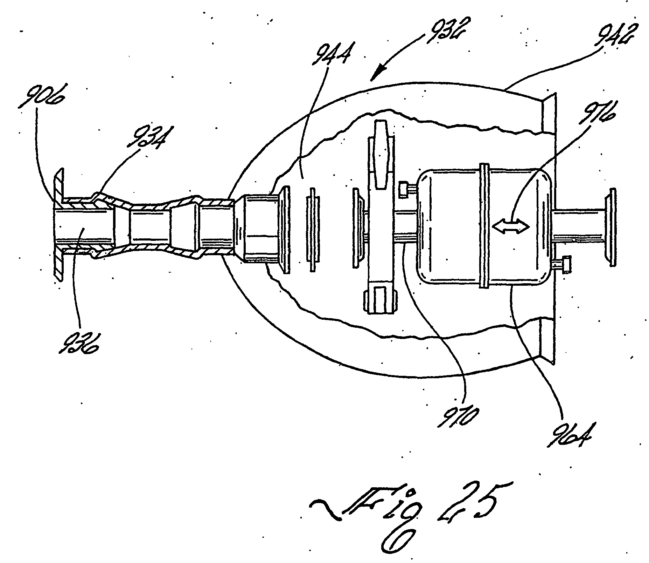

Fig. 25 is a top plan view of the second conduit system of Fig. 23 and a vent filter operatively connected to the second conduit system for controlling dust and/or degassing of the bioprocess container.

### DETAILED DESCRIPTION

Before proceeding with the description of the preferred embodiment, the following background will be helpful in understanding this invention.

### Filtration Background

Dynamic depth filtration, also known as pressure precoat filtration, is a constant flow, solid-liquid separation technique that has been practiced for over 75 years. Dynamic depth filtration uses a primary filter media that is a high purity, high-performance diatomite filter aid. Diatomite is a silica powder composed of diatoms, which are rigid, porous and irregular in shape, and Diatomite is essentially a dense, chert-like, consolidated diatomaceous earth.

Diatomaceous earth is well known in the art and is made of opaline shells of diatoms and has a consistency of dry powder. Diatomaceous earth has a wide variety of uses including, without limitation, uses as a filter aid, paint additive, sorbent, abrasive and thermal insulator. Diatomaceous earth is also known as kieselguhr and tripolite.

The use of the term "diatomaceous earth" as used herein is intended to cover all of the above-described materials.

In the field of filtration, many methods of particle separation from fluids employ diatomite products as filter aids. The intricate and porous structure unique to diatomite silica is particularly effective for the physical entrapment of particles in filtration processes. It is common practice to employ diatomite products when improving he clarity of fluids that contain suspended particles or particulate matter, or have turbidity.

Diatomire products are often applied to a septum to improve clarity and increase flow rate in filtration processes, in a step sometimes referred to as "precoating." Diatomite is also often added directly to a fluid as it is being filtered to reduce the loading or plugging of undesirable particulate at the septum while maintaining a designed liquid flow rate, in a step often referred to as "body feeding." Depending on the particular separation involved, diatomite products may be used in precoating, body feeding, or both. The principles involved with Diatomite filtration have been reviewed (Kiefer, 1991).

In some filtration applications, different diatomite products are blended together to further modify or optimize the filtration process. Also, diatomite products are sometimes combined with other substances. In some cases, these combinations may involve simple mixtures, for example, with cellulose, activated charcoal or carbon, clay, or other materials. In other cases, these combinations are composites in which diatomite products are intimately compounded with other ingredients to make sheets, pads, or cartridges. Still more elaborate modifications of any of these diatomite products are used for filtration or separation, involving, for example, surface treatment and the addition of chemicals to diatomite products, mixtures, or their composites.

In certain circumstances, diatomite products may also exhibit unique absorptive properties during filtration that can greatly enhance clarification or purification of a fluid. These adsorptive properties are highly specific, and depend upon weak forces for attraction of the absorbed species to weak electrical charges at the surface of diatomite, or upon the reactivity of silanol (i.e., =Si-OH) functional groups that often occur at the diatomite surface. For example, an ionized silanol group (i.e., =Si-0⁻) may react with a hydroniumion (i.e., H,0⁺) contributed by an acidic substance in solution, for example, citric acid (i.e. C₆H₈O₇), adsorbing the donated H⁺ at the surface in the Process.

In filtration applications, diatomic products are usually processed to provide a range of filtration rates that are closely related to their permeability reported in units of Darcies. Diatomic filter aids are available in a wide range of permeabilities. The selection of a filter aid with a particular permeability suitable for a specific process depends on the flow rate and degree of fluid clarification desired for the particular application.

Darcy's Law is well known to persons skilled in the art and is essentially an empirical law that governs flow through porous media and describes the relationship among flow rate, pressure drop and resistance. Functional filtration rates products are usually processed to provide a. range of filtration rates that are closely related to their permeability as reported in units of Darcies.

One Darcy correspond to the permeability through a filter media 1 cm thick which allows 1 cm³ of fluid with a viscosity of 1 centipoise to pass through an area of 1 cm² in 1 second under a pressure differential of 1 atmosphere.

Dynamic depth filtering is used in the beverage and food manufacturing industries, e.g., as a filter for beer, and in the medical industry, e.g., as a filter aid for human serum filtration.

One example is a pressure filter system used in the Currant Good Manufacturing Practices of the FDA ("CGMP") production of biopharmaceutical and other high purity products. In this CGMP production, a Celpure^{®} media is used for solid-liquid separation in a pressure filtration system. Celpure^{®} media is available in a wide range of permeabilities. The following Table A sets forth data for certain of the commercially available Celpure^{®} media:

**Table A**

| Grade | Permeability (Darcy) | Solids removed (micron)⁽¹⁾ |
|---|---|---|
| Celpure 65 | 0.065 | <0.3 |
| Celpure 100 | 0.100 | 0.3-0.45 |
| Celpure 300 | 0.300 | 0.45-0.6 |
| Celpure 1000 | 1.000 | 1-2 |

| | | |
|---|---|---|
| Note. (1) : Data is provided for comparison purposes only.. Depending on compressibility, of the solids, the values may range by more than an order of magnitude. Macroscopically, Celpure^{®} filter aids are characterized by their powdered form dry density (0.10 g/cm³ -0.40 g/cm³) and light colors that range from white to pink to gray. The maximum wet density specifications for Celpure^{®} media grade is about 0.3 g/cm³. | | |

A description of the solid-liquid separation with dynamic depth filtration is set forth in a Technical Bulletin entitled *Bench Scale Proof-of Principle with Celpure*^{®} Media, Technical Note AMC06, a publication of Advanced Minerals Corporation and World Minerals, Inc., Santa Barbara, California.

### Description of Bulk Mixing Apparatus for Forming a Solution of Dry Powder and Liquid

Typically, the suppliers of Diatomite comprising diatomaceous earth ship the material in bulk to an end user. The end user then mixes a predetermined volume of diatomaceous earth with a selected volume of process compatible solution, e.g. make-up water or feedstock solution, to form a solution comprising diatomaceous earth in suspension with a selected concentration, or selected solution density or when the solution density is within a density range of acceptable solution densities. The selected solution density and density range of the solution and batch mixing specifications are determined by the requirements of the end user.

The solution is then fabricated into or used as a diatomite filter media.

Typically, the batch mixing is performed by an end user customer using a large batch mixing apparatus. A typical batch mixing process will use about one (1) ton by weight of diatomaceous earth. The diatomaceous earth is transported into or loaded into a large batch mixing apparatus mixing vessel. A selected volume of a process compatible solution, e.g., make-up water, is then added to the diatomaceous earth. The ratio of process compatible solution to diatomaceous earth is dictated by the desired final selected concentration or density.

In this process, water from the local municipal water supply (which may or may not be filtered or preprocessed) is used as the process compatible solution. The large batch mixing apparatus then agitates the solution using mixing impellers or mixing blades for an appropriate mixing time, until the solution reaches the desired or selected concentration, density or a density in the density range. In this process, the selected concentration can be in the range of about 0.1 g/L to about 100 g/L.

The solution is then used to mold or fabricate the desired filter media for use as a filter.

### Description of Mixing Bag, Methods, Apparatus and System For Forming a Solution of Dry Powder and Liquid

The present invention has utility for improved handling of dry powder for forming solutions having dry powder in suspension with a selected concentration, generally, and specifically, for handling of diatomaceous earth for forming solutions having diatomaceous earth in suspension within a selected concentration wherein the selected concentration is at least one of a high concentration capable of being diluted to a target concentration and a target concentration intended for use without being diluted.

The solution can then be fabricated into a filter media that is an inert, high purity, high-performance diatomite filter as described above.

The method of the invention comprises the use of a mixing bag as shown in Fig. 1. In Fig. 1, the mixing bag 30 has an outer member having a predetermined shape and a side wall defining an interior mixing chamber shown generally by arrow 32 having a volume sufficient to receive a predetermined weight of dry powder and a selected volume of process compatible solution to form a solution having powder in suspension with a selected concentration. Typically, the selected concentration is specified by the individual user.

The end user may select the selected concentration to be a high concentration, e.g. a solution of Celpure^{®} media or LRA^{®} media at a concentration of 100 g/L to minimize the size of the processing system, and the resulting solution may then be diluted with an inline mixer to achieve a lower target concentration, e.g. 2 g/L.

Alternatively, the solution density may be used in lieu of concentration and a selected solution density in a range of solution densities acceptable to or specified by the end user. Typically, solution densities for the selected concentration solutions in the range of about 2g/L to about 100 g/L are in a range of about 0.5 g/cm³ to about 3 g/cm³, respectively.

The interior mixing chamber 32 has a first end 36 and a second end 38 wherein the second end 38 is located in an opposed spaced relationship to the first end 36.

The mixing bag 30 may be a preformed mixing bag having a selected shaped. In Fig. 1, the preformed mixing bag 30 has an outer member which is in the shape of an elongated thin walled rectangular member having a hollowed-out passageway defining the interior mixing chamber. In Fig. 1, the mixing bag 30 has a top section 42 and a bottom section 44. The first end 36 is located in the top section 42 and the second end 38 is located in the bottom section 44.

As shown in Figs 1 through 6, the mixing bag 30 has' at least three sealable openings 50,52 and 54.

The first sealable opening 50 extends from a location exterior to the mixing, bag 30 and into communication with the interior-mixing chamber 32 at the first end 36 of the top section 42. The first sealable opening 50 may be configured for at least one of enabling transporting dry powder into the interior mixing chamber 32, enabling agitation of the powder and process compatible solution to be transported into the interior mixing chamber 32 to form a solution having powder in suspension with a selected concentration, and enabling withdrawal of the solution at a selected concentration from the interior mixing chamber 32.

In this embodiment, the mixing bag 30 includes a third sealable opening 54 which is configured specifically for enabling transporting dry powder into the interior-mixing chamber 32. In this embodiment, the first sealed opening 50 is used for at least one of enabling agitation of the material and process compatible solution in the interior mixing chamber 32 to form a solution having the material in suspension with a selected concentration and enabling withdrawal of the solution at a selected concentration from the interior mixing chamber 32.

The second sealable opening 52 extends from a location exterior to the mixing bag 30 and into communication with the interior mixing chamber 32 at the second end 38 located in the bottom section 44. Opening 52 is configured.for at least one of injecting process compatible solution into the interior mixing chamber 32 to form a solution having powder in suspension, enabling agitation of the powder and process compatible solution in the interior mixing chamber 32 to form a solution having powder in suspension with a selected concentration and enabling withdrawal of the solution with a selected concentration from the interior mixing chamber 32.

The third sealable opening 54 extends from a location exterior to the mixing bag 30 and into communication with the interior-mixing chamber 32 at the first end 36 in top section 42. Opening 54 is configured for enabling transporting dry powder into the interior-mixing chamber 32. In the preferred embodiment, the structure of the sealable opening 54 includes a sanitary port assembly which is discussed in greater detail in the discussions of Fig. 14A and Fig. 14B.

In addition, the embodiment of Fig. 1 includes, as an optional opening, an auxiliary opening 60. Auxiliary opening 60 may be used during the process of fabricating a preformed mixing bag or could be configured to function as an air vent, degassing port for discharging biological gasses or as a vent check valve to enable expulsion or venting of gas of air from the mixing bag.

In the mixing bag 30, sealable opening 50, is configured to be sealed upon completion of transporting dry powder into the interior mixing chamber and is to be unsealed for enabling agitation of a powder and process compatible solution to form a solution having powder in suspension with a selected concentration and enabling withdrawal of the solution at said selected concentration from the interior mixing chamber 32.

In the mixing bag 30, sealable opening 52 is configured to be sealed sealed at least one of before commencement of transporting dry powder into the interior mixing chamber and after completion of transporting dry powder into the interior mixing chamber 32. Opening 52 is to be unsealed for at least one of enabling injection of a process compatible solution into the interior mixing chamber 32 to form a solution having powder in suspension, enabling agitation of a powder and process compatible solution to form a solution having powder in suspension with a selected concentration and withdrawal of solution from the interior mixing chamber with a selected concentration.

In the perspective pictorial representation of Fig. 2, a mixing bag 30 filled with a predetermined weight of dry powder 68 is shown as being placed in a shipping container 70 having a bottom member 72 and a recloseable top member 76 The shipping container 70 may be fabricated to have a preformed shipping support member formed in the bottom member 72 or, in the alternative, may include a separate insertable shipping support member which is placed in the bottom of the shipping container 70 prior to inserting of the mixing bag (filled or unfilled) into the shipping container 70. The preformed shipping support member formed in the bottom member 72 or the separate insertable shipping support member are shown by dashed lines 78 and typically is conical shaped member with the base positioned to receive and support the mixing bag 30 containing the dry powder 68. The shipping container 70 is used for shipping the preformed mixing bag 30 filled with a predetermined weight of dry powder 68 to an end user for subsequent injection of'a process compatible solution into the interior mixing chamber 32 to form a solution, also known as a slurry, having powder in suspension.

It is preferred that the preformed mixing bag 30 be placed into a shipping container 70 prior to the step of transporting dry powder 68 from a dry powder source through the first sealable opening 50, or in the alternative opening 54, into the interior mixing chamber 32 such that the mixing bag 30 has the dry powder 68 transported into the interior mixing chamber 32 when the mixing bag 30 is located within the shipping container 70.

Alternatively, the preformed mixing bag 30 can be placed into a shipping container 70 subsequent to the step of transporting dry powder. 68 from a dry powder source through the first sealable opening 50, or in the alternative opening 54, into the interior mixing chamber 32 such that the mixing bag 30 has the dry powder 68 in the interior mixing chamber 32 when the mixing bag is placed within the shipping container 70.

Figs. 3 through 6 show the structure of the preformed mixing bag 30 in greater detail.

In the embodiment of the mixing bag illustrated in Figs. 3 through 6, the preformed mixing bag 30 fabricated with three sealable openings, openings 50, 52 and 54. In such event, the sealable opening 54 is used for enabling transporting dry powder into the interior-mixing chamber 32. Sealable opening 52 is used for injecting a process compatible solution into the interior mixing chamber 32 to form a solution having powder in suspension, enabling agitation of the powder and process compatible solution in the inferior mixing chamber 32 to form a solution having powder in suspension with a selected concentration and enabling withdrawal of the solution with a selected concentration from the interior mixing chamber 32. The sealable opening 50 is used for enabling agitation of the powder and process compatible solution to be transported into the interior mixing chamber 32 to form a solution having powder in suspension with a selected concentration and enabling withdrawal of the solution at the selected concentration from the interior mixing chamber 32.

In the pictorial representation of Fig. 7, the mixing bag 30 of Figs. 1 through 6 is show as a preformed mixing bag 30 having a first sealable opening 50 and the third sealable opening 54 in the first end 36 and a second sealable opening 52 in the second end 38. The third sealable opening 54 is used for filling the interior mixing chamber 32 with a predetermined weight of dry material. Accordingly, the first sealable opening 50 is used for agitation of the solution and for enabling withdrawal of the solution at a selected concentration from the interior mixing chamber 32.

The second sealable opening 52 is used for infecting a process compatible solution into the interior mixing chamber 32 to form a solution having powder in suspension, enabling agitation of the powder and process compatible solution in the interior mixing chamber 32 to form a solution having powder in suspension with a selected concentration and enabling withdrawal of the solution with a selected concentration from the interior mixing chamber 32.

In Fig. 7, the first end 36 supports the first sealable opening 50 and the third opening 54. Opening 54 may be in the form of a port having a collar which terminates in'an orifice which can be sealed and unsealed as depicted in Fig. 14A and Fig. 14B. The first sealable opening may be fabricated using sealing techniques which are known to persons skilled in the art. In the present invention, conventional sanitary fittings are used which utilize a design that, when tightened, allow the gasket to extrude into the boreline of process piping and create a barrier that holds fluids. Examples of such a known sealable openings or sanitary fittings are Tri-Clamp^{®} brand sanitary fittings manufactured by Tri-Clover which comprises stainless steel components. Plastic disposable versions of sanitary fittings are available under the trademark SterilEnz Fittings through Paw BioScience Products.

Fig. 7, the second end 38 supports the second scalable opening 52. Sealable opening 52 may be fabricated using sealing techniques which are known to persons skilled in the art as described above.

During the step of transporting dry powder into the interior mixing chamber 32, the collar of the sealable opening 52 may become filled and possibly impacted by dry powder which could interfere with the step of injecting a process compatible solution through the salable opening arising from dry powder clogging the opening. This problem can be overcome by first tying off the collar from the exterior with a tying member 80 illustrated by dashed circle, e.g. rope, to collapse or restrict the collar of the sealable opening 52 to prevent the dry powder form becoming impacted in the collar. At the time of injecting a process compatible solution into the mixing bag 30, the tying member 80 is removed.

Fig. 7 illustrates a method for filling a mixing bag with a dry powder. The method comprises the steps of forming a mixing bag as described above, having an interior mixing chamber having a volume sufficient to receive a predetermined weight of dry powder and a selected volume of process compatible solution to form a solution having powder in suspension with a selected concentration.

In the pictorial representation of Fig. 8, the mixing bag 30 of Fig. 1 is show as a preformed mixing bag 30 having a first sealable opening 50, a second sealable opening 52 and a third sealable opening 54. A mixing control system 90 having a computer System 92 is used to control the method of using a preformed mixing bag 3 0 having the interior mixing chamber 32 filled with a predetermined weight of dry powder 68, shown in Fig. 2, to form a solution having powder in suspension with a selected concentration. The apparatus includes the mixing control system 90, a first conduit system 100, a second conduit system 102, a pump 106, a filtering system or filter 108 and clamps 110 and 112. The pump 106 is operatively connected to a source of process compatible solution 120 through a control valve 122. A concentration sensor or solution density sensor 130 may be used to monitor the concentration or solution density, respectively, of the solution during agitation. Solution may be withdrawn from the interior mixing chamber 32 through withdrawal member 154 or, alternatively a shown by dashed arrow 148. The filtering of the solution having powder in suspension through a filter 108 removes unwanted particles therefrom.

The computer system 92 can control the operation of: (i) the pump 106 through a control line shown by dashed line 138; (ii) the control valve 122 through a control line shown by dashed line 132; (iii) clamp 110 through :a control line shown by dashed line 134; and (iv) clamp 112 through a control line shown by dashed line 136. In addition, the filter 108 can be operated under computer control through a control line shown by dashed line 142. The sensor 130, which may monitor the concentration or solution density, can be operated under computer control through a control line shown by dashed line 7.46.

The term "computer" and "computer system" is intended to include all ancillary components such as, without limitation network servers, storage devices including rotating disk memory storage systems, modems, communication lines, digital subscriber linens ("DSL"), keyboards and the like.

The pictorial representation of Fig. 9 shows the step of injecting a selected volume of a process compatible solution from the source of process compatible solution 120 through the control valve 122 and pump 106 through the second conduit system 102 into a mixing bag 30 through opening 52. Control valve 110 is closed such that the pump 106 pressure is used to inject the process compatible solution through the second conduit system 102 and opening 52 in to the interior mixing chamber 32 having a. predetermined volume of dry powder.

The pictorial representation of Fig. 10 illustrates the steps of enabling agitation of the powder and process compatible solution to form a solution having powder in suspension with a selected concentration, filtering of the solution and for enabling withdrawal of the solution from the interior mixing chamber with a selected concentration under control of a mixing control system 90 of Fig. 8.

When the selected volume of process compatible solution is injected into the interior mixing chamber, the step of enabling agitation of the powder and process compatible solution in the interior mixing chamber 32 is commenced to form a solution having powder in suspension with a selected concentration. Clamp 110 is opened to permit passage of solution under pump pressure to pass through filter 108. Clamp 112 is closed to direct the solution through the inlet 150 of filter 108, through the filter 108 and out of the outlet 152. The solution flow is from the interior mixing chamber 32, out of opening 52, through the second conduit system 102, through the inlet 150, filter 108 and outlet 152, through the first conduit system 100, through opening 50-back into the interior mixing chamber 32.

When a determination is made that the solution comprising the dry powder in suspension in the process compatible solution is at a selected concentration or at a selected density, the step of withdrawal is illustrated in Fig. 10. The solution is selectively withdrawn from the interior mixing chamber 32 through either the opening 52 via the withdrawal member 154 having an orifice operatively connected to the opening 52 or conduit section 148 located adjacent the first opening 50.

Fig. 11 is a diagrammatic representation of the mixing bag 30 of the present invention having a filling conduit system 162 that is adapted for transporting dry powder into the interior mixing chamber through either first opening 50 or third opening 54 depending on the configuration of the preformed mixing bag 30.

In addition, Fig. 11 is a diagrammatic illustration of how the first conduit system 100 and a second conduit system 102 are operatively removeably connected to openings 50 and 52, respectively. The dashed lines 158 and 160 represent'flexible connecting hoses which operatively connect the first conduit system 100 to filter 108 and pump 106, shown in Fig. 8, and the second conduit system 102 to the pump 106. A controller 156 may be used for controlling the injecting make-up process compatible solution to inject a selected volume of process compatible solution into the interior mixing chamber. The controller may be the mixing control system 90 having a computer system 92 shown in Fig. 8 four controlling the method of using a preformed mixing bag 30 having the interior mixing chamber 32 filled with a predetermined weight of dry powder 68, shown in Fig. 2, to form a solution having powder in suspension with a selected concentration.

Fig. 12 is a top plan view of the first conduit system 100 of Fig. 11 illustrates that the first conduit system 100 is operatively removeably connected to opening 50 for enabling agitating the solution of powder and process compatible solution in the interior mixing chamber to form a solution of the process compatible solution having the powder in suspension with a selected concentration and enabling withdrawal of the solution with a selected concentration from the interior mixing chamber. The first conduit system 100 has a distal end 164 terminating in an insertion sleeve 166 that removeably couples to extension 168 of opening 50. The distal end 164 may be a flexible section to facilitate easy coupling of insertion sleeve 166 with extension 168.

Fig. 13 is a top plan view of the second conduit system 102 of Fig. 11 operatively removeably connected to opening 52 for at least one of injecting process compatible solution .into the interior mixing chamber to form a solution having powder in suspension, enabling agitation of the powder and process compatible solution in the interior mixing chamber to form a solution having powder in suspension with a selected concentration and enabling withdrawal of the solution with a selected concentration from the interior mixing chamber.

The second conduit system 102 has a distal end 170 terminating in an insertion sleeve 174 which removeably couples to extension 176 of opening 52. The distal end 170 may be a flexible section to facilitate easy coupling of insertion sleeve 174 with extension 176 of opening 52.

In Figs. 12 and 13, the exposed proximal ends of the first conduit system 100 and the second conduit system 102 are enclosed or sealed with a sealing enclosure 178 to prevent contamination.

Fig. 14A is an exploded front elevational view of the third sealable opening 54 in the form of a sanitary port or sanitary fitting assembly and the assembly is shown generally as sealing assembly 182. The sealing assembly 182 is used for enabling transporting a predetermined weight of dry powder into the interior mixing chamber 32 of a mixing bag 30. The sealing assembly 182 includes a collar section 180 having a sealing rim 184 which is operatively connect to the opening 54. A sealing washer 186 is positioned between the sealing rim 184 and a sealing cover 188. A port securing member 192 has an adjustable threaded member 194 to permit the port securing member 192 is be positioned around the sealing rim 184 and enclose the sealing washer 186 and sealing cover 188. The adjustable threaded member 194 is then tightened to cause the port-securing member to form a seal of the opening 54. The sealing assembly 182 permits easy sealing and unsealing of opening 54. The same sealing assembly 182 could likewise be used for openings 50 and 52.

Fig.. 14B is an exploded front elevational view of the third sealable opening 54 in the form of a sanitary port or sanitary fitting assembly having a filing extension and the assembly is shown generally as sealing assembly 196. The sealing assembly 196 is used for enabling transporting a predetermined weight of dry powder into the interior mixing chamber 32 of a mixing.bag 30. The sealing assembly 196 includes a collar section 180 having a sealing rim 184 which is operatively connect to the opening 54. A sealing washer 186 is positioned between the sealing rim 184 and a filing extension housing 198 having a mating washer surface 200 for sealably engaging the sealing washer 186 A port securing member 192 has an adjustable threaded member 194 to permit the port securing member 192 to be positioned around the sealing rim 184 and mating washer surface 200 of a filing extension housing 198 enclosing the sealing washer 186. The adjustable threaded member 194 is then tightened to form a seal of the opening 54. The sealing assembly 182 permits easy sealing and unsealing of opening 54.

Fig. 15 is a pictorial representation of yet another embodiment of a preformed mixing bag 300 for practicing the teachings of the present invention. The preformed mixing bag 300 has a first sealable opening 350, a second sealable opening 352 and a third sealable opening 360. The mixing bag 302 has a magnetically controlled impeller 406 located in the interior-mixing chamber 380 which is used for agitating the solution. The magnetically controlled impeller 406 is magnetically coupled to a magnetic driving member 402 which is driven by shaft 400.

In the pictorial representation of Fig. 16, the magnetically controlled impeller 406, located in the interior mixing chamber 380, is illustrated as being magnetically coupled to the magnetic driving member 402 via magnetic lines of force 412 by rotation of shaft 400 driven by motor 410. Such an agitation apparatus may be used for selected volumes of dry powder in excess of 20 kg.

The word "agitation" as used herein is used in its broadest sense and is intended to include all known apparatus and systems for mixing powder and fluids or liquids to form a solution including, without limitation, pumps, magnetic mixing apparatus, mechanical vibration apparatus, mechanical mixing apparatus wherein a mixing apparatus may be inserted through a opening to mix the solution, mechanical mixing apparatus having impellers driven by a shaft and motor or the like.

Fig. 17 is a block diagram of the method performed usually performed at a first location, typically the location of the manufacturer or distributor, for filling a preformed mixing bag having an interior mixing chamber configured to have a volume sufficient to receive a predetermined weight of dry powder and a selected volume of process compatible solution to form a solution having powder in suspension with a selected concentration and shipping the same in a shipping container to an end user. A preformed mixing bag which has been formed and which is ready to fill is represented by block 500. The appropriate opening is unsealed as represented by block 502. The mixing bag is filled with a selected volume of dry powder as represented by block 504. The filled mixing bag with the dry powder is then shipped to an end user in a shipping container as represented by arrow 520.

Fig. 18 is a block diagram of the method, usually performed at a second location, typically the location of the end user, four using a preformed mixing bag having an interior-mixing chamber containing a predetermined weight of dry powder. The appropriate sealable openings are unsealed as shown by element 522 for enabling injection of a process compatible solution, e.g. a liquid, into the interior mixing chamber as shown by element 524 to form a solution having powder in suspension, enabling agitation of the powder and process compatible solution to form a solution having powder in suspension with a selected concentration likewise depicted by element 524 and enabling withdrawal of the solution with a selected concentration as depicted by element 526.

The solution may be in the form of a filter aid which can be used in a dynamic filtering application as described hereinbefore.

Fig. 19 is a pictorial representation of another embodiment of a preformed mixing bag 600 wherein the outer member is in the shape of an elongated thin walled cylindrical member 602 having a hollowed-out passageway 604 defining the interior-mixing chamber 604 in the form of an internal conical shaped hopper 662 for receiving dry powder. The mixing bag has three sealable openings, 650, 652 and 660. Opening 660 is used for enabling transporting dry powder into the interior-mixing chamber formed by hopper 662. Opening 652 is used for at least one of injecting a process compatible solution into the interior mixing chamber to form a solution having powder in suspension, enabling agitation of the powder and process compatible solution in the interior mixing chamber to form a solution having powder in suspension with a selected concentration and enabling withdrawal of the solution with a selected concentration from the interior mixing chamber 604 in the form of an internal conical shaped hopper 662. opening 650 is used for enabling agitation of the powder and process compatible solution in the interior mixing chamber to form a solution having powder in suspension with a selected concentration. This operations of the first conduit system 672 and second conduit system 674 are under control of a mixing control system 670, having a computer system 92 shown in Fig. 8, to form a solution having powder in suspension with a selected concentration.

In the embodiment represented by. Fig. 20, a preformed mixing bag 700 is used in a system for the delivery of Celpure^{®} media to a filtration process wherein the suspension, slurry or solution is in the form of a Celpure^{®} media body-feed concentration produced with a process compatible solution.

The mixing bag 700 has an interior mixing chamber 702 has a volume sufficient to receive a predetermined weight of dry material capable of being forming and remaining in suspension in a process compatible solution and a selected volume of process compatible solution to form a solution having the material in suspension with a selected concentration. A sealable opening 704 is provided for separately filling the mixing bag 700 with a dry powder at a remote location as described in Fig. 17. The mixing bag 700 may be filled with a selected volume of Celpure^{®} media, e.g., Celpure^{®} 300 or Celpure^{®} 1000 offered for sale and sold by . , World Minerals, Inc., Santa Barbara; California.

The preformed mixing bag 700 has a substantially rectangular shape and has three sealable opening, 704, 706 and 708. Opening 704 is dedicated as a filling port. Openings 706 and 708 function as the at least two sealable openings as described herein before.

Appropriately sized peristaltic pumps depicted by elements 722, 724 and 726 are provided to perform the functions of injecting a process compatible solution into the interior mixing chamber 702 of mixing bag 700, agitating the solution and withdrawing the solution when a selected concentration is achieved.

Line 730 extends from opening 708 to pump 720. Line 734 extends from opening 706 and is bifurcated to extend through a clamp 744 to pump 722 and to branch line 740 to clamp 748. Line 752 extends from clamp 748 to line 760 which extends between pump 720 and clamp 766. Line 770 extends between a source of feedstock or process compatible solution 772. Line 774 is connected to line 770 and extends to a clamp 776 located at the inlet of pump 724. Line-800 extends from pump 724 to line 802. Line 802 connects pump 722 to a filter 804 to perform the dynamic filtering of a designated fluid to be filtered.

In operation, the mixing bag having the dry powder is placed into position and the apparatus is connected as described. The mixing bag may contain 5 kg of Celpure^{®} media which is to be used for the delivery of 5kg of Celpure^{®} media to a filtration process. The mixing bag holds up to 50L of suspension or solution which will produce a Celpure^{®} body feed concentration of 100 g/L. The suspension may be produced with a process compatible solution, e.g., water, or feedstock, e.g. whole yeast cells, mammalian cells and Lysed E. Coli cells.

Depending on the required body feed rate, the solution at a selected concentrated may be pumped, either undiluted at 100 g/L, into the filter 804 or diluted with feedstock, via an inline mixer pump 724, to a lower target concentration, e.g. 2g/L.

The mixing bag 700 is injected or filled with a process compatible fluid from source 772 using pump 720. Clamp 776 is closed to occlude line 774 and clamp 766 is opened providing the pump 720 with access to the source 772 through line 760, opened clamp 766 and line 770. The volume of process compatible solution would be about 48L. The pump 720 fills the mixing bag' at about 20L/min.

Upon completion of the injecting of the process compatible solution into the mixing bag 700, the agitation step is then initiated. This is accomplished by closing clamp 766, opening clamp 748 and reversing flow of pump 720 at a flow rate of about 20L/min. The agitation time period is about 10 minutes to obtain the selected concentration of 100g/L.

Upon completion of the step of agitating the solution, the solution is ready to be withdrawn. The withdrawal is accomplished by opening clamp 744 to withdraw the solution at the selected concentration from line 740 using pump 722 through line 802 to the filter 804.

If it is desired to reduce the higher concentration to a lower target concentration using a feed stock form source 772, the following steps may be taken. Clamp 766 is occluded or closed and clamp 776 is open to enable pump 724 to controllably dilute the solution with the feed stock at an appropriate feed rate to obtain the desired lower target concentration.

Depending on the structure of the mixing bag, the mixing bag can be recycled for subsequent cycle, or if the mixing bag is a single use-mixing bag, the mixing bag is then discarded.

The teachings of this invention include use of the mixing bag in a system. The system comprises a mixing bag having an interior-mixing chamber having a volume sufficient to receive a predetermined weight of dry powder and a selected volume of process compatible solution to form a solution having powder in suspension with a selected concentration and wherein the interior mixing chamber has a predetermined weight of dry powder stored therein. The interior-mixing chamber having a first end and a second end wherein the second end is located in an opposed spaced relationship to the first end. The mixing bag includes three sealable openings as indicated above

The system includes a first conduit system 100 configured to be operatively removeably connected to the first sealable opening for enabling agitating of the solution of powder and process compatible solution in the interior mixing chamber to form a solution of the process compatible solution having the powder in suspension with a selected concentration. A second conduit system 102 is operatively removeably connected to the second sealable opening and to the first conduit system for enabling injection of a process compatible solution into the interior mixing chamber through the second opening to form a solution having powder in suspension, for enabling agitation of the powder and process compatible solution within the interior mixing chamber through the second opening to form a solution having powder in suspension with a concentration and for enabling withdrawal of solution from the interior mixing chamber with a selected concentration through the second opening.

A pump 106 shown in Fig. 11 is operatively connected to the first conduit system 100 and to the second conduit system 102. The pump 106 is configured to at least one of injecting process compatible solution from a source of process compatible solution into.the interior chamber to form a solution having powder in suspension and agitating the solution of powder and process compatible solution within interior mixing chamber to form a solution having powder in suspension with a selected concentration.

Withdrawal of the solution at the selected concentration may be performed using at least one of the first conduit system and the second conduit system as discussed hereinbefore.

The system may further comprise a controller for controlling the pump to inject a selected volume of make-up process compatible solution from a source of process compatible solution into the interior-mixing chamber.

### Operating Examples

### EXAMPLES

The following are examples are based on use of a preformed mixing bag having an interior mixing chamber having a volume sufficient to receive a predetermined weight of dry powder and a selected volume of process compatible solution to form a solution having powder in suspension with a selected concentration according to the method of the present invention. In certain of the Examples set forth below, the Examples include use of Celepure P65^{®} media, Celepure 300^{®} media and Celepure 1000^{®} media. The specifications of the above Celpure^{®} media are set forth below for reference.

### Celepure P65^{®} media

Celepure P65^{®} media is characterized as a Lightly Calcined, Purified Siliceous Earth, the specifications of which are as follows:

| Specification Properties | Minimum | Maximum |
|---|---|---|
| Vel permeability (millDarcy | 40 | 80 |
| Centrifuged wet density (g/cm³) | **-** | 0.26 |
| PH (-Log [H]) | 5 | 8 |
| Conductivity (µS/cm) | - | 15 |
| Acid soluble iron (ppm) | - | 8 |
| Citric acid ethanol soluble aluminum (ppm) | - | 8 |
| Endotoxin | - | 1 |

| National Formulary | Maximum |
|---|---|
| Moisture 105°C (%) | 0.5 |
| Ignition loss-dry (%) | 2.0 |
| Acid solubles (%) | 2.0 |
| Water-soluble substances (%) | 0.2 |
| Acid soluble iron (ppm) | 10 |
| Acid soluble iron lead (ppm) | 10 |
| Non-siliceous substances (mg) | 50 |

### Celepure P100^{®} media

Celepure P100^{®} media is characterized as a Calcined, Purified Siliceous Earth, the specifications of which are as follows:

| Specification Properties | Minimum | Maximum |
|---|---|---|
| Vel permeability (millDarcy | 70 | 140 |
| Centrifuged wet density (g/cm³) | - | 0.26 |
| PH (-Log [H]) | 5 | 8 |
| Conductivity (µS/cm) | - | 15 |
| Acid soluble iron (ppm) | - | 8 |
| Citric acid ethanol soluble aluminum (ppm) | - | 6 |
| Endotoxin - | - | 1 |

| National Formulary | Maximum |
|---|---|
| Moisture 105°C (%) | 0.5 |
| Ignition loss-dry (%) | 2.0 |
| Acid solubles (%) | 2.0 |
| Water-soluble substances (%) | 0.2 |
| Acid soluble iron (ppm) | 10 |
| Acid soluble iron lead (ppm) | 10 |
| Non-siliceous substances (mg) | 50 |

### Celepure P300^{®} media

Celepure P300^{®} media is characterized as a Lightly Calcined, Purified Siliceous Earth, the specifications of which are as follows:

| Specification Properties | Minimum | Maximum |
|---|---|---|
| Vel permeability (millDarcy | 150 | 300 |
| Centrifuged wet density (g/cm³) | - | 0.25 |
| PH (-Log [H]) | 5 | 8 |
| Conductivity (µS/cm) | - | 15 |
| Acid soluble iron (ppm) | - | 5 |
| Citric acid ethanol soluble aluminum (ppm) | - | 4 |
| Endotoxin | - | 1 |

| National Formulary | Maximum |
|---|---|
| Moisture 105°C (%) | 0.5 |
| Ignition loss-dry (%) | 2.0 |
| Acid solubles (%) | 2.0 |
| Water-soluble substances (%) | 0.2 |
| Acid soluble iron (ppm) | 10 |
| Acid soluble iron lead (ppm) | 10 |
| Non-siliceous substances (mg) | 50 |

### Celepure P1000^{®} media

Celepure P1000^{®} media is characterized as a Lightly Calcined, Purified Siliceous Earth, the specifications of which are as follows:

| Specification Properties | Minimum | Maximum |
|---|---|---|
| Vel permeability (millDarcy | 750 | 1,250 |
| Centrifuged wet density (g/cm³) | - | 0.24 |
| PH (-Log [H]) | - | 8.8 |
| Conductivity (µS/cm) | - | 15 |
| Acid soluble iron (ppm) | - | 5 |
| Citric acid ethanol soluble aluminum (ppm) | - | 4 |
| Endotoxin | - | 1 |

| National Formulary | Maximum |
|---|---|
| Moisture 105°C (%) | 0.5 |
| Ignition loss-dry (%) | - 2.0 |
| Acid solubles (%) | 2.0 |
| Water-soluble substances (%) | 0.2 " |
| Acid soluble iron (ppm) | 10 |
| Acid soluble iron lead (ppm) | 10 |
| Non-siliceous substances (mg) | 50 |

### EXAMPLE 1

The following example utilizes Celepure 100^{®} media.

A preformed mixing bag having a structure illustrated in Fig. 1 and formed of a HyClone HyQ CX5-14 Film was used. The mixing bag had three (3) sealable openings. A tying member was affixed to the collar of the second sealable opening to prevent impaction of the dry powder in the collar.

Five kilograms (5 kg) of Celepure 100^{®} media was then transported into the mixing bag through the third sealable opening in the form of a sealing port shown in Fig. 14A. The third sealable opening was sealed. The first sealable opening and the second sealable opening were sealed thereby confining the 5 kg of dry powder in the interior-mixing chamber of the mixing bag.

The solution was prepared using the five kilograms (5 kg) of Celepure P100^{®} media stored in the mixing bag. The tying member affixed to the collar of the second sealable opening was removed. The second sealable opening and third sealable opening were unsealed. The first conduit system and the second conduit system were then operatively attached the first sealable opening and the second sealable opening, respectively. The make-up process compatible solution was injected through the second sealable opening. The total selected volume of process compatible solution injected into the interior mixing chamber was about 13 gallons (about 48 liters). The injection rate was about 5.3 gallons per minute (about 20L/minute). Upon injection of the selected volume of liquid into the interior-mixing chamber, a peristaltic pump was used to provide the desired agitation through the second and third sealable openings. The reversible pump agitation was at the rate of about 3 gallons per minute (about 10L/minute) with size .75 inch ID tubing. The filter was a standard process compatible solution filter to remove impurities and had a through put capability equal to the pump flow rate.

The time period for agitation was about ten (10) minutes.

In this example, the solution was agitated to form a solution having dry powder in suspension with a selected concentration of 100g/L.

The solution was then withdrawn and used as a filter aid as discussed in connection with Fig. 20 above.

### EXAMPLES 2 THROUGH 4

Table B below set forth Examples 2, 3 and 4 using the method of the present invention and the system and apparatus described in Fig. 20 above.

**TABLE B**

| **DESCRIPTION OF PROCESS VARIABLES** | **MIXING BAG FILLED WITH PROCESS COMPATIBLE SOLUTION & DILUTED WITH FEEDSTOCK** | | |
|---|---|---|---|
| **Feedstock Type** | **EXAMPLE 2 Whole Yeast Cells** | **EXAMPLE 3 Mammalian Cells (1% Viability)** | **EXAMPLE 4 Lysed *E. Coli Cells*** |
| Feedstock Volume in MIXING BAG (L) | NA | NA | NA |
| Feedstock Volume outside MIXING BAG (L) | 400 | 400 | 200 |
| Process Compatible Solution Type | Buffer | Buffer | Buffer |
| Process Compatible Solution Vol (L) | 95.31 | 95.31 | 190.61 |
| Media in MIXING BAG | LRA | Celpure 300 | Celpure P65 |
| Media Quantity in MIXING BAG (kg) | 10.000 | 10.000 | 20.000 |
| MIXING BAG Capacity (L) | 100 | 100 | 200 |
| MIXING BAG Media Concentration (g/L) | 100.0 | 100.0 | 100.0 |
| Filtration Body Feed (g/L) | 20.0 | 20.0 | 50.0 |
| Required Dilution Rate | 0.2 | 0.2 | 0.5 |
| Required Flux Rate (L/m²-min) | 5.0 | 5.0 | 2.5 |
| Withdrawing Pump [Draws from MIXING BAG] (L/min) | 1.00 | 1.00 | 1.25 |
| Inline Dilution Pump [Draws from Feedstock and/or Process Compatible Solution] (Lmin) | 4.00 | 4.00 | 1.25 |

### EXAMPLES 5 THROUGH 8

Table C below set forth Examples 5, 6, 7 and 8 using the method of the present invention and the system and apparatus described in Fig. 20 above.

**TABLE C**

| **DESCRIPTION OF PROCESS VARIABLES** | **MIXING BAG FILLED WITH FEEDSTOCK** | | | |
|---|---|---|---|---|
| **Feedstock Type** | **EXAMPLE 5 Whole Yeast Cells** | **EXAMPLE 6 Whole Yeast Cells** | **EXAMPLE 7 Mammalian Cells (1% Viability)** | **EXAMPLE 8 Lysed *E. Coli Cells*** |
| Feedstock Volume in MIXING BAG (L) | 49.95 | 49.95 | 95.31 | 190.61 |
| Feedstock Volume .outside MIXING BAG (L) | 0 | 0 | 400 | 200 |
| Process Compatible Solution Type | NA | NA | NA | NA |
| Process Compatible Solution Vol (L) | NA | NA | NA | NA |
| Media in MIXING BAG | LRA | Celpure P1000 | Celpure P300 | Celpure P65 |
| Media Quantity in MIXING BAG (kg) | 0.100 | 0.100 | 10.000 | 20.000 |
| MIXING BAG Capacity (L) | 50 | 50 | 100 | 200 |
| MIXING BAG Media Concentration (g/L) | 2.0 | 2.0 | 100.0 | 100.0 |
| Filtration Body Feed (g/L) | 2.0 | 2.0 | 20.0 | 50.0 |
| Required Dilution Rate | 1.0 | 1.0 | 0.2 | 0.5 |
| Required Flux Rate (L/m²-min) | 10.0 | 10.0 | 5'.0 | 2.5 |
| Withdrawing Pump [Draws from MIXING BAG] (L/min) | 10.0 | 10.0 | 1.00 | 1.25 |
| Inline Dilution Pump [Draws from Feedstock and/or Process Compatible Solution] (Lmin) | 0.00 | 0.00 | 4.00 | 1.25 |

### Mixing Bag Materials

Packaging configurations for the mixing bag used in the method of the present invention and materials filled therein for practicing this invention come in a variety of sizes for non-bulk options. The preformed mixing bag can be fabricated to be a round shape, square shape, rectangular shape, oblong shape, conical shape, elliptical shape, triangular shaped or other geometrical shape. The bottom of the mixing bag can be fabricated to be slopped, conical shaped, flat or other appropriate shape to support the dry powder in the mixing bag.

The mixing bag, when placed into a shipping container, assumes the shape of the container. As discussed above with reference to Fig. 2, the shipping container may be fabricated to include a preformed shipping support member formed in the bottom member thereof or, in the alternative, may include a separate insertable shipping support member which is placed in the bottom of the shipping container prior to inserting of the mixing bag (filled or unfilled) into the shipping container. As such, a preformed shipping support member formed in the bottom member or a separate insertable shipping support member functions to provide a means for enabling the mixing bag and dry powder to assume the shape of the holder.

Such non-bulk options include mixing bags formed of plastic materials, poly materials, Hyclone films, e.g. HyQ CX5 Film, or other appropriate chemically and physically compatible materials, as is well known to persons skilled in the art. For Example, in pharmaceutical applications, the weights of dry powder could be in the 5 kg to 15 kg range of weights of dry powder using a mixing bag formed of poly materials and the weights of dry powder weights in the range of 10 kg to 22.5 kg range of weights of dry powder using a mixing bag formed of HyClone films, e.g. 3-web Film, HYQM1 and HYQBX6 (Attane 4203), a single web film, HyQCX5-9, Formulation PL2403 and a single web Film, HyQCX5-14, Formulation PL2438-1.

Sterile Fluid handling bags for medical applications and mixing bags for industrial and other applications as discussed above are typically constructed of multi-layer film(s). The following are typical materials utilized in the manufacture of multi-layer films suitable for use in mixing bags and are shown in Table D:

**Table D**

| Identification of Layer | Characteristic of Layer |
|---|---|
| Fluid Contact Layer | Offers excellent |
| Ultra Low Density Polyethylene | biocompatibility as well as chemical compatibility with a wide range of solutions. |
| (ULDPE) | |
| Gas Barrier Layer | Provides outstanding performance as a barrier to atmospheric gasses. |
| Ethyl Vinyl Alcohol | |
| (EVOH) | |
| Strength Layer | Offers excellent strength properties for bags that have. the potential to be used or manipulated outside a cylindrical tank or tote |
| Nylon | |
| (NYLON) | |
| Tie Layer | Materials provide outstanding layer fusion. Materials contribute to overall bag strength and gas barrier properties. USP<88> Class VI materials |
| Ethyl Vinyl Acetate and/or Linear Low Density Polyethylene | |
| (TIE) | |

The following are examples of multi-layer bags that can be fabricated from the above materials and are shown in Table E:

**Table E**

| Material A | Application | | Description |
|---|---|---|---|
| | Sterile Fluid | Layer 1-NYLON | |
| | Handling Bags | Layer 2-TIB | |
| | | Layer 3-EVOH | |
| | | Layer 4-TIE | |
| | | Layer 5-ULDPE | |
| B | Sterile Fluid | Layer 1-NYLON | Inner Film Layers |
| | Handling Bags | Layer 2-TIE | |
| | | Layer 3-EVOH | |
| | | Layer 4-TIE | |
| | | Layer 5-ULDPE | Outer Film Layers |
| | | Layer 6-TIE | |
| | | Layer 7-ULDPE | |
| | | Layer 8-ULDPE | |
| C | Large | Layer 1-TIE | |
| | Container Bags | Layer 2-ULDPE | |
| | | Layer 3-ULDPE | |
| D | Cassette Bags | Layer 1-ULDPE | Inner Film |
| | | Layer 2-ULDPE | Barrier Film |
| | | Layer 3-ULDPE | Outer Film Layers |
| | | Layer 4-EVOH | |
| | | Layer 5-ULDPE | |

The mixing bags used in the method of the present invention may comprise single layer film or multilayer films depending on the application and engineering requirements for the bag. The materials used for fabricating the mixing bag can be selected to maximize performance of the mixing bag.

Topically, the mixing bags are designed for single use applications. An Article entitled Quantitative *Economic Evaluation of Single Use Disposables in BioProcess*, comprising nine (9) pages, which appeared in the May/June, 2002 issue of Pharmaceutical Engineering, compares single use technology and traditional steel vessels used in biomanufacturing as well as an introduction to the concept of disposable bag technology.

### Utility And Product Attributes Of The Invention

By utilizing the teachings of the present invention, there are three (3) important dry powder material, referred to as the product, aspects which remain substantially constant in the solution and during the process of forming the solution. The three (3) important dry powder material product aspects are as follows: (i) the solution maintains substantially the same product density when used in an application, e.g., as a filter cake when the solution is used as a filter aid; (ii) the permeability of the product in solution remains substantially unchanged; and (iii) the integrity of the product in solution remains substantially the same, e.g., the structure of the material product does not substantially decompose or break down into smaller particles during formation of the solution.

One attribute of forming a solution using a selected dry material using, the teachings of the present invention, is that the process of forming the solution, including agitation thereof, does not significantly degrade the critical physical characteristics of the dry powder material product which is essential in an application using the solution which relies on the critical physical characteristics of the material product in solution remaining substantially unchanged to perform a desired function.

In one application, the dry material used may be a Celpure^{®} Diatomite that is to be used as a filter aid. In such application, the fundamental intricate, fine particulate, and porous structure of the diatom frustule forming the Celpure^{®} Diatomite gives diatomite its low density, high surface area, high porosity and permeability. These attributes give diatomite its unique commercial value and versatility unmatched by other natural forms of silica for certain applications, e.g. in filtration applications. Moreover diatomite may be processed to impart a specific permeability range which is of general importance in solids-liquids separation applications.

In the field of filtration, many methods of particle separation from fluids employ diatomite products as filter aids. The intricate and porous structure unique to diatomite silica is particularly effective for the physical entrapment of particles in filtration processes. It is common practice to employ diatomite products when improving the clarity of fluids that contain suspended particles or particulate matter, or have turbidity.

For use in filtration, diatomite products are usually processed to provide a range of filtration rates, which are closely related to their permeability. Permeability is reported in units of Darcies, as discussed hereinbefore, and is commonly abbreviated "Da". The permeability, in Da, is readily determined (European Brewery Convention, 1987) using a specially constructed device designed to form a filter cake on a septum from a suspension of diatomite in water, and then measuring the time required for a specified volume of water to flow through a measured thickness of.filter cake of known cross-sectional area. The principles have been previously derived for porous media from Darcy's law (Bear, 1972), so an array of alternative devices and methods are in existence that correlate well with permeability. Diatomite filter aids that are currently commercially available span a wide range of permeability, from about 0.001 Da to about 30 Da. The selection of a filter aid with a particular permeability suitable for a specific filtration process depends on the flow rate and degree of fluid clarification desired for the particular application. As such, using a dry powder with a known or selected permeability which remains substantially unchanged during preparation of the solution using the dry powder in accordance with the teachings of this invention is one of the desired attributes of this invention.

In another application, the dry material used may be LRA^{®}, Celpure^{®}, and Chromosorb^{®} designed to be used as Sorbents and Chromatography Media. In such application, a sorbent's usefulness is in removing soluble constituents from biologic and non-biologic solutions and the desired criteria for using the same can be traced to its surface area, density, or other physical attributes that effect the mass transfer of solutes in solution.

As such, using a dry powder with known or selected physical attributes that effect the mass transfer of solutes in solution and a dry powder with known or selected physical attributes which remain substantially unchanged during preparation of the solution using the dry powder in accordance with the teachings of this invention is one of the desired attributes of this invention.

### Other Applications

It is envisioned that the mixing bag interior mixing chamber has a volume sufficient to receive a predetermined weight of dry material capable of being forming and remaining in suspension in a process compatible solution and a selected volume of process compatible solution to form a solution, suspension or slurry having the material in suspension with a selected concentration or select density or a density within a range of selected densities. Such a mixing bag has utility for a variety of applications. The dry material can be a granulated or powder concentrate capable of forming a solution. It is preferable that the powder be as dry as possible. However, the teachings of the present invention can be used with a slightly hydrated media. A more wet or the heavier hydrated media makes it more difficult it becomes to break up the particles to form the desired solution, suspension or slurry. Injecting of the process compatible solution from the bottom of the mixing bag enhances the formation of the desired solution, suspension or slurry, particularly with a dry medium or a slightly hydrated media.

The mixing bag can be used in the industrial field for producing solutions used in making filter aids; in the agriculture field for fertilizers, pesticides, gypsum; in the medical field for kidney dialysis and in the food and beverage industry for making solutions from dry powder for use as beverages or the like. For example, United States Patent 5,121,857 discloses agitating apparatus for dispensing fruit juices from a bag in a box arrangement wherein the a metalized plastics material flexible bag containing a fruit drink having fruit particles suspended therein. The mixing bag of the present invention can be used with such agitating and dispensing apparatus in the beverage industry.

The dry powder is a non-soluble powder. In the preferred use of the teachings of the present invention, the non-soluble powder maybe be selected from a group consisting of diatomaceous earth, Celepure P65^{®} media, Celepure P100^{®} media, Celepure 300^{®} media, Celepure P1000^{®} media, LRA^{®} media and sorbents.

The dry powder may be a Sorbent. A Sorbent is associated with Sorption. Sorption is the association of a gas phase or aqueous substance with a solid material, known as the sorbent, by surface attachment (adsorption) or dissolution and migration into the solid phase (absorption). "Solid phase" refers to material that behaves as a solid according to macroscale measures. At a chemical level, the surface of that solid may act more like an oil film. "Adsorption" refers to association of a sorbate with surfaces. "Absorption" implies penetration of a sorbate into the sorbent**,** by diffusion.

The dry powder can be used for Chromatography. By classical definition, chromatography is a separation process that is achieve by distributing the substances to be separated between a mobile phase and a stationary phase. Those substances distributed preferentially in the moving phase pass through the chromatographic system faster than those that are distributed preferentially in the stationary phase. As a consequence, the substances are eluted from the column inverse order of their distribution coefficients with respect to the stationary phase. In this definition LRA^{®}, Celpure^{®}, and Chromosorb^{®} can all be called chromatography media, sorbents, or filter aids.

LRA^{®} is not a diatomite, however it.is a silicate. Chromosorb products are a mix. Some are solely diatomite, some are diatomite coated with polymers (e.g. Teflon) and some are pure polymers.

### Celpure is 100% diatomite

The teachings of the present invention has utility for the hydrating of chromatography media in a mixing bag and the withdrawal of and loading of chromatography media at a concentrated solution from the mixing bag into chromatography media columns in the same manner as a Celepure^{®} media is used as a filter aid as described herein.

It is also envisioned that if a significant volume of dry powder is to be simultaneously processed with a process compatible solution to form a solution, suspension or slurry having a selected concentration, that a plurality of mixing bags could be concurrently injected with a process compatible solution, agitated using an agitation process, apparatus or system disclosed herein and the resulting solution at selected concentration can then be withdrawn either sequentially or in parallel from the plurality of mixing bags.

It will be appreciated that various alterations and modifications may be made to the method of the invention and to the mixing bag used therein. All such variations and modifications should be considered to fall within the scope of the invention as broadly hereinbefore described and as claimed hereafter.

### Bioprocess Container for Pharmaceutical Applications

In the pictorial representation of Fig. 21 another embodiment or species of a preformed mixing bag used in the method of the invention in the form of a bioprocess container 900 configured for use in pharmaceutical applications is shown. The bioprocess container 900 has three sealable inlets or openings 904, 906 and 908. The three sealable inlets or openings 904, 906 and 908 could be used to inject a process compatible fluid (or a feedstock solution or make-up water) to be processed to form the solution, for agitating the solution and/or withdrawing the solution from the bioprocess container, according to the method of the invention. In this embodiment, sealable inlet or opening 906 is used to fill the interior mixing chamber of the bioprocess container 900 having a volume sufficient to receive a predetermined weight of substantially dry material capable of remaining in suspension in a process compatible solution and a selected volume of process compatible solution to form a solution having the material in suspension with a selected concentration.
If desired, a feed stock solution or fluid could be injected into the interior chamber of the bioprocess container through sealable inlet or opening 904.

In this embodiment, sealable inlet or opening 908 is used for at least one of injecting a process compatible fluid to be processed to form the solution, agitating the solution of powder and process compatible solution in the interior mixing chamber to form a solution of the process compatible solution having the powder in suspension with a selected concentration and/or for withdrawal of the solution with a selected concentration from the interior mixing chamber of the bioprocess container 900.

A first conduit system 912, shown in Figs. 21 and 22, is operatively removeably connected to the sealable inlet or opening 904 for injecting a feedstock solution, if desired, and/or agitating the solution of powder and process compatible solution in the interior mixing chamber to form a solution of the process compatible solution having the powder in suspension with a selected concentration in the presence of a feed stock material.

The first conduit system 912 has a distal end 920 terminating in an insertion sleeve 924 that removably couples to extension 926 of opening 904. The distal end 920 may be a flexible section to facilitate easy coupling of insertion sleeve 924 with extension 926.

A second conduit system shown in Fig. 23 is operatively removeably connected to sealable inlet or opening 906 for enabling filling of a dry powder into the interior of the bioprocess mixing bag.

A closeable bag or cover 928 protects a coupling member depicted by arrow 930 during storage or shipment.

The second conduit system 932 has a distal end 934 terminating in an insertion sleeve 936 which removeably couples to extension 938 of opening 906. A closeable bag or cover 942 protects a coupling member depicted by arrow 944 during storage or shipment.

Fig. 24 is a top plan view of a third conduit system 948 shown in Fig. 21 and the third conduit system 948 is operatively removeably connected to sealable inlet or opening 908 for at least one of injecting process compatible solution into the interior mixing chamber to form a solution having powder in suspension, for enabling agitation of the powder and process compatible solution in the interior mixing chamber to form a solution having powder in suspension with a selected concentration and in cooperation with the first conduit system and/or enabling withdrawal of the solution with a selected concentration from the interior mixing chamber of the bioprocess container 900.

The third conduit system 948 has a distal end 950 terminating in an insertion sleeve 952 which removeably couples to extension 956 of opening 908. A closeable bag or cover 960 protects a coupling member depicted by arrow 962 during storage or shipment.

Fig. 25 is a top plan view of the second conduit system 932 of Fig. 23 and a vent filter 964 operatively connected to the second conduit system 932 for controlling dust and/or degassing of the bioprocess container 900. The vent filter has a first end 970 which is configured to cooperate with the connecting member 944 such that fluid flow is in the direction of into the interior chamber as shown by arrow 976.

The vent filter may have a filter size of about .7 microns to about 5 microns for a bioprocess application.

In use, the bioprocess container has an interior-mixing chamber configured to receive a predetermined weight of dry powder, e.g. a predetermined volume of diatomaceous earth. Sealable inlet or opening 906 is preferably used for filling the interior chamber with a predetermined volume of dry material.

Sealable inlet or opening 908 is used to inject the process compatible fluid and/or a feedstock solution into the interior mixing chamber and sealable openings 904 at 908 are used to agitate the solution using a pump or other means as discussed above in connection with Fig. 8, 9 and 10.

When ths solution comprising the dry powder, e.g. diatomaceous earth, and process compatible fluid reaches a suspension with a selected concentration, or selected solution density or a solution density which is within a density range of acceptable solution densities, the sealable inlet or opening 908 opening is used for withdrawing solution with a selected concentration, or selected solution density or a solution density which is within a density range of acceptable solution densities. The selected solution density and density range of the solution' and batch mixing specifications are determined by the requirements of the end user.

The material selected for the bioprocess container preferably would have the following characteristics:
(1) Biological Reactivity: The standard product contact materials must pass current USP Class VI (USP <88>) and Cytotoxicity (USP <87> MEM Elution) testing.
(2) Physiochemical: The standard product contact materials must pass USP Physiochemical Tests for Plastics (USP <661>).
(3) Endotoxin: The material used for the bioprocess should be tested for the presence of endotoxin per the USP Bacterial Endotoxin Test (USP <85>) and for any container aqueous extracts being < 0.25 EU/ml as determined by the Limulus Amebocyte Lysate Test (LAL);
(4) Particulate: The material used for the bioprocess should be tested for particulate per Particulate Matter in Injections Light Obscuration Particulate Count Test (USP <788>); and
(5) Sterility: The material used for the bioprocess should be tested for sterility following The Association for the Advancement of Medical Instrumentation (AAMI) guidelines. Quarterly validation has determined that an irradiation dose of 25 - 38 kGy provides a minimum Sterility Assurance Level (SAL) of 10⁻⁶.

All of the numerical and quantitative measurements set forth in this application (including in the description, claims, abstract, drawings, and any appendices) are approximations.

The invention illustratively disclosed or claimed herein suitably may be practiced in the absence of any element which is not specifically disclosed or claimed herein. Thus, the invention may comprise, consist of, or consist essentially of the elements disclosed or claimed herein.

The following claims are entitled to the broadest possible scope consistent with this application- The claims shall not necessarily be limited to the preferred embodiments or to the embodiments shown in the examples.

## Claims

1. A method comprising the use of a mixing bag (30) having an interior mixing chamber (32) having a first end (36) located in a top section (42) and a second end (38) located in a bottom section (44), and having a predetermined weight of dry powder material, the dry powder material being non-soluble in a process compatible solution, said mixing bag (30) having:
a first sealable opening (50) extending from a location exterior to the mixing bag (30) and into communication with the interior mixing chamber (32) at said first end (36) and which is configured for at least one of enabling transportation of the material into the interior mixing chamber (32), enabling agitation of the material and process compatible solution in the interior mixing chamber (32) to form a solution having the material in suspension with a selected concentration and enabling withdrawal of the solution with a selected concentration from the interior mixing chamber (32);
a second sealable opening (52) extending from a location exterior to the mixing bag (30) and into communication with the interior mixing chamber (32) at said second end (38) and which is configured for at least one of enabling injection of the process compatible solution into the interior mixing chamber (32) to form a solution having the material in suspension with a selected concentration, enabling agitation of the material and process compatible solution in the interior mixing chamber (32) to form a solution having the material in suspension with a selected concentration and enabling withdrawal of the solution with a selected concentration from the interior mixing chamber (32); and
a third sealable opening (54) extending from a location exterior to the mixing bag (30) and into communication with the interior mixing chamber (32) at said first end (36) and which is configured for enabling transporting the dry powder material into the interior mixing chamber (32),
the method comprising the steps of:
injecting a selected volume of the process compatible solution into the mixing bag (30) through the second sealable opening (52);
agitating through at least one of said first and second sealable openings (50, 52) the material and process compatible solution in the interior mixing chamber (32) to form a solution having material in suspension with a selected concentration; and
withdrawing from the interior mixing chamber (32) through at least one of said first and second sealable openings (50, 52) the solution with a selected concentration.

2. The method of claim 1 wherein said third sealable opening (54) is in the form of a sanitary filling port.

3. The method of claim 1 wherein said dry powder material is a diatomaceous earth.

4. The method of claim 1 wherein said dry powder material is selected from the group consisting of diatomaceous earth, Celpure P65^{®} media, Celpure P100^{®} media, Celpure 300^{®} media, Celpure F1000^{®} media, LRA^{®} media and sorbents.

5. The method of claim 1 further comprising the step of connecting said second sealable opening (52) to a source of a process compatible solution.

6. The method of claim 1 wherein the step of agitating through at least one of said first and second sealable openings (50, 52) the material and process compatible solution in the interior mixing chamber (32) to form a solution having the material in suspension with a selected concentration further includes filtering through a filter (108) the solution having material in suspension to remove unwanted particles therefrom.

7. The method of claim 1 further comprising the step of:
removing the mixing bag (30) having the dry powder material in the interior mixing chamber (32) from a shipping container (70).

8. The method of claim 1 wherein the step of removing the mixing bag (30) having the dry powder material in the interior mixing chamber (32) from a shipping container (70) is prior to the step of injecting a selected volume of a process compatible solution through said second sealable opening (52) to form a solution having the material in suspension with a selected concentration.

## Patentansprüche

1. Verfahren, umfassend den Einsatz eines Mischbeutels (30) mit einer innenliegenden Mischkammer (32), die ein erstes Ende (36) in einem oberen Abschnitt (42) und ein zweites Ende (38) in einem unteren Abschnitt (44) hat, und der ein vorgegebenes Gewicht trockenes pulverförmiges Material enthält, wobei das trockene pulverförmige Material in einer prozesskompatiblen Lösung unlöslich ist und der Mischbeutel (30) aufweist:
eine erste verschließbare Öffnung (50), die sich von einer Stelle außerhalb zum Mischbeutel (30) erstreckt und die in Verbindung steht mit der innenliegenden Mischkammer (32) am ersten Ende (36), und die ausgelegt ist für mindestens eine Verwendung, ausgewählt aus: Erlauben eines Transports des Materials in die innenliegende Mischkammer (32), Erlauben eines Bewegens des Materials und der prozesskompatiblen Lösung in der innenliegenden Mischkammer (32), so dass man eine Lösung vorgegebener Konzentration erhält, worin das Material in Suspension ist, und Erlauben eines Entziehens der Lösung vorgegebener Konzentration aus der innenliegenden Mischkammer (32);
eine zweite verschließbare Öffnung (52), die sich von einer Stelle außerhalb zum Mischbeutel (30) erstreckt und die in Verbindung steht mit der innenliegenden Mischkammer (32) am zweiten Ende (38), und die ausgelegt ist für mindestens eine Verwendung, ausgewählt aus: Erlauben eines Einspritzens der prozesskompatiblen Lösung in die innenliegende Mischkammer (32) so dass man eine Lösung vorgegebener Konzentration mit dem Material in Suspension erhält, Erlauben eines Bewegens des Materials und der prozesskompatiblen Lösung in der innenliegenden Mischkammer (32), so dass man eine Lösung vorgegebener Konzentration mit dem Material in Suspension erhält, und Erlauben eines Entziehens der Lösung vorgegebener Konzentration aus der innenliegenden Mischkammer (32); und
eine dritte verschließbare Öffnung (54), die sich von einer Stelle außerhalb zum Mischbeutel (30) erstreckt und die in Verbindung steht mit der innenliegenden Mischkammer (32) am ersten Ende (36), und die ausgelegt ist für das Erlauben eines Transports des trockenen pulverförmigen Materials in die innenliegende Mischkammer (32);
wobei das Verfahren die Schritte umfasst:
Einspritzen eines gewählten Volumens der prozesskompatiblen Lösung durch die zweite verschließbare Öffnung (52) in den Mischbeutel (30);
Bewegen des Materials und der prozesskompatiblen Lösung in der innenliegenden Mischkammer durch mindestens die erste und/oder zweite Öffnung (50, 52), so dass eine Lösung des Materials in der vorgegebenen Konzentration gebildet wird; und
Abziehen der Lösung vorgegebener Konzentration aus der innenliegenden Mischkammer (32) durch mindestens die erste oder zweite verschließbare Öffnung (50, 52).

2. Verfahren nach Anspruch 1, wobei die dritte verschließbare Öffnung (54) als Hygieneeinfüllöffnung ausgebildet ist.

3. Verfahren nach Anspruch 1, wobei das trockene pulverförmige Material Diatomeenerde ist.

4. Verfahren nach Anspruch 1, wobei das trockene pulverförmige Material ausgewählt ist aus der Gruppe Diatomeenerde, Celpure P65^{®}-Medium, Celpure P100^{®}-Medium, Celpure 300^{®}-Medium, Celpure P1000^{®}-Medium, LRA^{®}-Medium und Sorptionsmittel.

5. Verfahren nach Anspruch 1, zudem umfassend das Verbinden der zweiten verschließbaren Öffnung (52) mit einer Quelle einer prozesskompatiblen Lösung.

6. Verfahren nach Anspruch 1, wobei das Bewegen des Materials und der prozesskompatiblen Lösung in der innenliegenden Mischkammer (32) zum Bereitstellen einer Lösung vorgegebener Konzentration mit dem Material in Suspension durch mindestens die erste oder die zweite verschließbare Öffnung (50, 52) zudem das Filtrieren der Lösung mit dem Material durch Filter (108) umfasst, um unerwünschte Teilchen daraus zu entfernen.

7. Verfahren nach Anspruch 1, zudem umfassend das Entfernen des Mischbeutels (30) mit dem trockenen pulverförmigen Material in der innenliegenden Mischkammer (32) aus einem Transportbehälter (70).

8. Verfahren nach Anspruch 1, wobei das Entfernen des Mischbeutels (30) mit dem trockenen pulverförmigen Material in der innenliegenden Mischkammer (32) aus dem Transportbehälter (70) vor dem Einspritzen des gewählten Volumens prozesskompatibler Lösung durch die zweite verschließbare Öffnung (52) erfolgt, so dass man eine Lösung der gewählten Konzentration erhält, worin das Material in Suspension ist.

## Revendications

1. Procédé comprenant l'utilisation d'un sac de mélange (30) comportant une chambre de mélange intérieure (32) présentant une première extrémité (36) située dans une section supérieure (42) et une seconde extrémité (38) située dans une section inférieure (44) et ayant un poids prédéterminé de matériau pulvérulent sec, le matériau pulvérulent sec n'étant pas soluble dans une solution compatible avec le traitement, ledit sac de mélange (30) ayant :
une première ouverture scellable (50) s'étendant à partir d'un emplacement extérieur au sac de mélange (30) et en communication avec la chambre de mélange intérieure (32) au niveau de la première extrémité (36) et configurée pour au moins l'une des fonctions suivantes : permettre le transport du matériau dans la chambre de mélange intérieure (32), permettre l'agitation du matériau et d'une solution compatible avec le traitement dans la chambre de mélange intérieure (32) pour former une solution ayant le matériau en suspension avec une concentration sélectionnée, et permettre le retrait de la solution ayant une concentration sélectionnée hors de la chambre de mélange intérieure (32) ;
une deuxième ouverture scellable (52) s'étendant à partir d'un emplacement extérieur au sac de mélange (30) et en communication avec la chambre de mélange intérieure (32) au niveau de ladite seconde extrémité (38) et configurée pour au moins l'une des fonctions suivantes : permettre l'injection de la solution compatible avec le traitement dans la chambre de mélange intérieure (32) pour former une solution ayant le matériau en suspension avec une concentration sélectionnée, permettre l'agitation du matériau et de la solution compatible avec le traitement dans la chambre de mélange intérieure (32) pour former une solution ayant le matériau en suspension avec une concentration sélectionnée, et permettre le retrait de la solution ayant une concentration sélectionnée de la chambre de mélange intérieure (32) ;
une troisième ouverture scellable (54) s'étendant à partir d'un remplacement extérieur au sac de mélange (30) et en communication avec la chambre de mélange intérieure (32) au niveau de ladite première extrémité (36) et configurée pour permettre le transport du matériau pulvérulent sec dans la chambre de mélange intérieure (32),
le procédé comprenant les étapes consistant à :
injecter un volume sélectionné de la solution compatible avec le traitement dans le sac de mélange (30) par la deuxième ouverture scellable (52) ;
agiter, à travers au moins l'une desdites première et deuxième ouvertures scellables (50, 52) le matériau et la solution compatible avec le traitement dans la chambre de mélange intérieure (32) pour former une solution ayant du matériau en suspension avec une concentration sélectionnée ; et
retirer de la chambre de mélange intérieure (32), à travers au moins l'une desdites première et deuxième ouvertures scellables (50, 52), la solution ayant une concentration sélectionnée.

2. Procédé selon la revendication 1, dans lequel ladite troisième ouverture scellable (54) se présente sous la forme d'un orifice d'alimentation sanitaire.

3. Procédé selon la revendication 1, dans lequel ledit matériau pulvérulent sec est une terre de diatomées.

4. Procédé selon la revendication 1, dans lequel ledit matériau pulvérulent sec est choisi dans le groupe constitué par la terre de diatomées, un milieu de Celpure P65^{®}, un milieu de Celpure P100^{®}, un milieu de Celpure P300^{®}, un milieu de Celpure P1000^{®}, un milieu de LRA^{®}, et des sorbants.

5. Procédé selon la revendication 1, comprenant en outre l'étape consistant à relier ladite deuxième ouverture scellable (52) à une source d'une solution compatible avec le traitement.

6. Procédé selon la revendication 1, dans lequel l'étape consistant à agiter à travers au moins l'une desdites première et deuxième ouvertures scellables (50, 52), le matériau et la solution compatible avec le traitement dans la chambre de mélange intérieure (32) pour former une solution ayant le matériau en suspension avec une concentration sélectionnée comprend en outre le filtrage à travers un filtre (108) de la solution ayant le matériau en suspension afin d'en éliminer les particules indésirables.

7. Procédé selon la revendication 1, comprenant en outre l'étape consistant à :
enlever le sac de mélange (30) comportant le matériau pulvérulent sec dans la chambre de mélange intérieure (32) d'un conteneur de transport (70).

8. Procédé selon la revendication 1, dans lequel l'étape consistant à enlever le sac de mélange (30) comportant le matériau pulvérulent sec dans la chambre de mélange intérieure (32) d'un conteneur de transport (70) est antérieure à l'étape consistant à injecter un volume sélectionné d'une solution compatible avec le traitement par ladite deuxième ouverture scellable (52) pour former une solution ayant le matériau en suspension avec une concentration sélectionnée.
